# EUROPEAN PATENT APPLICATION

(11) **EP 1 741 788 A1**
(43) Date of publication of application: **10.01.2007**
(21) Application number: 05090199.0
(22) Date of filing: 06.07.2005
(51) Int. Cl.: C12N 15/82, A01H 5/00

(54) **Transgenic plant with tissue-selectively reduced cytokinin receptor activity**

(71) Applicant: Freie Universität Berlin, 14195 Berlin (DE)
(72) Inventor: Riefler, Michael, Dr., 14195 Berlin (DE); Schmülling, Thomas, Dr. Prof., 14195 Berlin (DE)
(74) Representative: Jungblut, Bernhard Jakob

(57) **Abstract**

The invention relates to a transgenic plant, wherein the activity of AHK2- and/or AHK3- DNA, RNA and/or protein is selectively suppressed or reduced in a specific target tissue of the plant, and wherein the activity of AHK2- and AHK3-DNA, RNA and protein is essentially unaltered in at least one tissue not being target tissue, to methods of making such plants, to methods of making seeds of enhanced size and/or plants with enhanced root branching, and uses of such plants.

### Annex to the application documents - subsequently filed sequences listing

SEQUENCE LISTING

<110> Freie Universität Berlin
Riefler, Michael
Schmülling, Thomas

<120> Transgenic plant with tissue-selectively reduced
cytokinin-receptor activity

<130> FUB/EP/0501

<140> EP 05090199.0
<141> 2005-07-06

<160> 50

<170> PatentIn version 3.3

<210> 1
<211> 9
<212> PRT
<213> Artificial

<220>
<223> Chase domain consensus sequence


<220>
<221> MISC_FEATURE
<222> (3)..(3)
<223> X is A or G

<220>
<221> misc_feature
<222> (7)..(7)
<223> Xaa can be any naturally occurring amino acid

<400> 1

Cys Lys Xaa Val Leu Thr Xaa Pro Phe
1               5


<210> 2
<211> 7
<212> PRT
<213> Artificial

<220>
<223> Chase domain consensus sequence


<220>
<221> misc_feature
<222> (4)..(5)
<223> Xaa can be any naturally occurring amino acid

<220>
<221> MISC_FEATURE
<222> (7)..(7)
<223> X is F or Y

<400> 2

Leu Gly Val Xaa Xaa Thr Xaa
1               5

<210> 3
<211> 20
<212> DNA
<213> Artificial

<220>
<223> Primer

<400> 3
ttaatattat gggaaggagt 20


<210> 4
<211> 21
<212> DNA
<213> Artificial

<220>
<223> Primer

<400> 4
tttccctcaa tgcaataacc t 21


<210> 5
<211> 21
<212> DNA
<213> Artificial

<220>
<223> Primer

<400> 5
tctgccattg atcagagaac a 21


<210> 6
<211> 20
<212> DNA
<213> Artificial

<220>
<223> Primer

<400> 6
tccccaattt ctgagccata 20


<210> 7
<211> 20
<212> DNA
<213> Artificial

<220>
<223> Primer

<400> 7
gctatgaggg tgctccattc 20


<210> 8
<211> 20
<212> DNA
<213> Artificial

<220>
<223> Primer

<400>  8
cgcatctcat gctttctcaa                                                    20


<210>  9
<211>  20
<212>  DNA
<213>  Artificial

<220>
<223>  Primer

<400>  9
tggagtggct tatgctgaaa                                                    20


<210>  10
<211>  20
<212>  DNA
<213>  Artificial

<220>
<223>  Primer

<400>  10
aacggtattg gtgccttttg                                                    20


<210>  11
<211>  21
<212>  DNA
<213>  Artificial

<220>
<223>  Primer

<400>  11
tctgccattg atcagagaac a                                                  21


<210>  12
<211>  20
<212>  DNA
<213>  Artificial

<220>
<223>  Primer

<400>  12
tccccaattt ctgagccata                                                    20


<210>  13
<211>  22
<212>  DNA
<213>  Artificial

<220>
<223>  Primer

<400>  13
gctatgaggg tgtgctccat tc                                                 22


<210>  14
<211>  20
<212>  DNA
<213>  Artificial

<220>
<223>  Primer

<400>  14
cgcatctcat gctttctcaa                                                    20


<210>  15
<211>  20
<212>  DNA
<213>  Artificial

<220>
<223>  Primer

<400>  15
tggagtggct tatgctgaaa                                                    20


<210>  16
<211>  20
<212>  DNA
<213>  Artificial

<220>
<223>  Primer

<400>  16
aacggtattg gtgccttttg                                                    20


<210>  17
<211>  31
<212>  DNA
<213>  Artificial

<220>
<223>  Primer

<400>  17
ggggacaagt ttgtacaaaa aagcaggctg g                                       31


<210>  18
<211>  30
<212>  DNA
<213>  Artificial

<220>
<223>  Primer

<400>  18
ggggaccact ttgtacaaga aagctgggtg                                         30


<210>  19
<211>  21
<212>  DNA
<213>  Artificial

<220>
<223>  Primer

<400>  19
tctgccattg atcagagaac a                                                  21

<210>  20
<211>  20
<212>  DNA
<213>  Artificial

<220>
<223>  Primer

<400>  20
tccccaattt ctgagccata                                                    20


<210>  21
<211>  20
<212>  DNA
<213>  Artificial

<220>
<223>  Primer

<400>  21
gctatgaggg tgctccattc                                                    20


<210>  22
<211>  20
<212>  DNA
<213>  Artificial

<220>
<223>  Primer

<400>  22
cgcatctcat gctttctcaa                                                    20


<210>  23
<211>  20
<212>  DNA
<213>  Artificial

<220>
<223>  Primer

<400>  23
tggagtggct tatgctgaaa                                                    20


<210>  24
<211>  20
<212>  DNA
<213>  Artificial

<220>
<223>  Primer

<400>  24
aacggtattg gtgccttttg                                                    20


<210>  25
<211>  21
<212>  DNA
<213>  Artificial

<220>
<223>  Primer

<400>  25
tctgccattg atcagagaac a                                                21

<210>  26
<211>  20
<212>  DNA
<213>  Artificial

<220>
<223>  Primer

<400>  26
tccccaattt ctgagccata                                                  20

<210>  27
<211>  20
<212>  DNA
<213>  Artificial

<220>
<223>  Primer

<400>  27
gctatgaggg tgctccattc                                                  20

<210>  28
<211>  20
<212>  DNA
<213>  Artificial

<220>
<223>  Primer

<400>  28
cgcatctcat gctttctcaa                                                  20

<210>  29
<211>  20
<212>  DNA
<213>  Artificial

<220>
<223>  Primer

<400>  29
tggagtggct tatgctgaaa                                                  20

<210>  30
<211>  20
<212>  DNA
<213>  Artificial

<220>
<223>  Primer

<400>  30
aacggtattg gtgccttttg                                                  20

<210>  31
<211>  20
<212>  DNA
<213>  Artificial

<220>
<223>  Primer

<400>  31
agggcatcag gaaaaggagt                                                    20


<210>  32
<211>  20
<212>  DNA
<213>  Artificial

<220>
<223>  Primer

<400>  32
tgtctgtttg ctggcaagtt                                                    20


<210>  33
<211>  20
<212>  DNA
<213>  Artificial

<220>
<223>  Primer

<400>  33
ggtatctcgg gggagtgttt                                                    20


<210>  34
<211>  20
<212>  DNA
<213>  Artificial

<220>
<223>  Primer

<400>  34
cgcatctcat gctttctcaa                                                    20


<210>  35
<211>  20
<212>  DNA
<213>  Artificial

<220>
<223>  Primer

<400>  35
aagagcgtat tgcagccact                                                    20


<210>  36
<211>  20
<212>  DNA
<213>  Artificial

<220>
<223>  Primer

<400>  36
ccaaaatcga gcttgctctc                                                    20

<210> 37
<211> 20
<212> DNA
<213> Artificial

<220>
<223> Primer

<400> 37
agggcatcag gaaaaggagt 20


<210> 38
<211> 20
<212> DNA
<213> Artificial

<220>
<223> Primer

<400> 38
ccaaaatcga gcttgctctc 20


<210> 39
<211> 20
<212> DNA
<213> Artificial

<220>
<223> Primer

<400> 39
agggcatcag gaaaaggagt 20


<210> 40
<211> 20
<212> DNA
<213> Artificial

<220>
<223> Primer

<400> 40
ccaaaatcga gcttgctctc 20


<210> 41
<211> 20
<212> DNA
<213> Artificial

<220>
<223> Primer

<400> 41
ttaatattat gggaaggagt 20


<210> 42
<211> 21
<212> DNA
<213> Artificial

<220>
<223> Primer

<400>  42
tttccctcaa tgcaataacc t                                                21

<210>  43
<211>  21
<212>  DNA
<213>  Artificial

<220>
<223>  Primer

<400>  43
tctgccattg atcagagaac a                                                21

<210>  44
<211>  20
<212>  DNA
<213>  Artificial

<220>
<223>  Primer

<400>  44
tccccaattt ctgagccata                                                  20

<210>  45
<211>  20
<212>  DNA
<213>  Artificial

<220>
<223>  Primer

<400>  45
gctatgaggg tgctccattc                                                  20

<210>  46
<211>  20
<212>  DNA
<213>  Artificial

<220>
<223>  Primer

<400>  46
cgcatctcat gctttctcaa                                                  20

<210>  47
<211>  40
<212>  DNA
<213>  Artificial

<220>
<223>  Sequence of the T-DNA Insertion site

<400>  47
ctgaatttct tcagctgaat atattgtggt gtaaacaaat                            40

<210>  48
<211>  45
<212>  DNA
<213>  Artificial

<220>
<223>  Sequence of the T-DNA Insertion site

<400>  48
ctttgagagg ccacttacta agcacattga cgcttagaca actta                    45


<210>  49
<211>  40
<212>  DNA
<213>  Artificial

<220>
<223>  Sequence of the T-DNA Insertion site

<400>  49
tttgttcttc agaaaatatt caggatatat tgtggtgtaa                          40


<210>  50
<211>  52
<212>  DNA
<213>  Artificial

<220>
<223>  Sequence of T-DNA Insertion site

<400>  50
ttagaagaca agaagatgcg acggaatatg gagaaatcta catggatcag ca            52

## Description

### Field of the invention.

The invention relates to a transgenic plant with reduced cytokinin-receptor activity, a process for making such a plant, uses of such a plant, and a method for enhancing seed size and/or root branching.

Background of the invention and state of the art.

Cytokinin is a plant hormone that plays positive and negative regulatory roles in many aspects of plant growth and development. It stimulates the formation and activity of shoot meristems, is able to establish sink tissues, retard leaf senescence, inhibits root growth and branching, and plays a role in seed germination and stress responses. Analysis of cytokinin-deficient plants has shown that cytokinin plays opposite roles in shoot and root meristems and suggests that the hormone has an essential function in quantitative control of organ growth. In Arabidopsis the cytokinin signal is perceived by three sensor histidine kinases, AHK2, AHK3 and AHK4. These three receptors show a high degree of sequence identity, but each has distinguishing characteristics. The current model of cytokinin signaling predicts that the receptors feed into the two-component signaling system of Arabidopsis. The activated receptors autophosphorylate conserved histidine residues in their kinase domains. The phosphoryl residue is then transferred to an Asp residue of the receiver domain. Subsequently the signal is transported via phosphorelay to a phosphotransmitter shuttle protein which moves to the cell nucleus where it activates B-type response regulators. B-type response regulators function as transcriptional activators. Among their target genes are A-type response regulator genes. One function of A-type response regulators is to attenuate the cytokinin response as negative feedback regulation. Furthermore, A-type response regulators may link cytokinin signaling with other signaling pathways.

mRNA of all three receptor genes is found in all organs, although with different abundance. AHK4 is predominantly expressed in the root, where its mRNA was mainly localized to the vascular cylinder and the pericycle of the root. Gel blot analyses and promoter-GUS fusions have detected AHK4 expression in different shoot tissues, although in lower abundance than in the roots. The AHK2 and in particular the AHK3 gene show greater expression in the aerial parts of Arabidopsis plants.

The publications Ch. Nishimura et al., The Plant Cell, 16:1365-77 (2004) and M. Higuchi et al., PNAS 101(23):8821-25 (2004) disclose AHK2/AHK3 double mutants of Arabidopsis thaliana, wherein these cytokinin receptors are non-functional. The seedling phenotype showed a decreased hypocotyl length, wherein this effect was largely due to a reduction of cell number, but not to the cell size. Further, the plants showed compact rosettes with shortened petioles and small leaf blades. The leafs expanded slower than the wild type controls. Effects on root branching and seed size are not reported.

The publication T. Werner et al., PNAS, 98(18):10487-492 (2001) discloses a transgenic Nicotiana tabacum plant, wherein cytokinin oxidase is overexpressed, which degrades cytokinins. Cytokinin deficiency resulted in diminished activity of the vegetative and floral shoot apical meristems and leaf primordia. By contrast, cytokinins are negative regulators of root growth and lateral root formation.

The publications WO 01/96580 A2, US 2003/0074698 A1 and US 2005/0044594 A1 disclose plants, wherein cytokinin oxidase is overexpressed resulting in larger seeds and roots, wherein, however other phenotype features are of disadvantange. Root branching is not addressed in detail.

Technical problem of the invention.

The technical problem underlying the instant invention is to provide a method for making seeds of enhanced size and plants with enhanced root branching, thereby, however, avoiding disadvantageous phenotype features.

Summary of the invention and preferred embodiments.

The present invention teaches a transgenic plant, wherein the activity of at least one cytokinin-receptor naturally occurring in the plant is selectively suppressed or reduced in a specific target tissue of the plant, and wherein the activity of the cytokinin-receptor is essentially unaltered in at least one tissue of the plant not being target tissue.

Preferably two, three, up to all cytokinin-receptors of the plant are selectively suppressed or reduced in the target tissue.

Cytokinin-receptors are known for several plants. Cytokinin-receptors of Arabidopsis are in particular AHK2, AHK3 and AHK4. Depending on the purpose, different combinations of cytokinin-receptors may be suppressed or reduced. E.g. in Arabidopsis suppression or reduction of AHK2, AHK3 and AHK4 is favorable, if enhanced seed size is to be achieved. If enhanced root branching is to be achieved, AHK2 and AHK3 only may be suppressed or reduced. Generally, depending on the specific plant, the optimum combination of cytokinin-receptors to be suppressed or reduced may be identified by simple experiments.

Plant cytokinin receptor genes other than Arabidopsis have been published by e.g. in Yonekura-Sakakibara et al., Plant Physiol. 134, 1654-1661 (2004). Presently unknown cytokinin-receptors in plants may, in addition, be identified by detection of the so-called CHASE domain. The CHASE domain of AHK4 has on the protein level 70% homology with the closest homologous sequence, which is found in rice. Homology to one HK of maize is 68%, to Arabidopsis AHK3 60%, and to AHK2 59%. There are other related domains with homologies in the range of 30-50%. According to the invention short peptide sequences have been identified, which are conserved in all known plant CHASE domains and are diagnostic for cytokinin receptors. These are:
1. CKa/gVLTxPF
2. LGVxxTf/y
wherein x can be any amino acid, a/g and f/y means that these amino acids occur with high probability, all other amino acids are invariable. Thus, the invention also teaches the use of an isolated peptide or protein comprising the sequence CKnVLTxPF, wherein n is A or G and x is any amino acid, or comprising the sequence LGVxxTm, wherein m is F or Y and x has then meaning as above, or of an isolated nucleic acid coding for such a protein or peptide for making a cytokinin-receptor inhibitor, wherein a substance, preferably a small molecule, antibody, peptide, protein, or inhibitory nucleic acid is constructed by means of molecular modeling, which binds to said amino acid sequence or to said nucleic acid sequence, or wherein such substance is selected from a substance pool after subjecting the substances of the substance pool to a binding assay detecting binding to said amino acid sequence or nucleic acid sequence and having detected binding substances as well as the use of an isolated peptide or protein comprising the sequence CKnVLTxPF, wherein n is A or G and x is any amino acid, or comprising the sequence LGVxxTm, wherein m is F or Y and x has then meaning as above, or of an isolated nucleic acid coding for such protein or peptide for identifying a cytokinin-receptor in a plant, wherein nucleic acids or proteins naturally occurring in the plant are screened for partial sequences being similar or identical with one of said sequences and wherein a nucleic acid or a protein is selected, which comprises a partial sequence with at least 80%, preferably 100%, identity with one of said sequences, as being cytokinin-receptor-functional. The grade of sequence identity (sequence matching) may be calculated using e.g. BLAST, BLAT or BlastZ. Preferably, sequence matching analysis may be supplemented by established homology mapping techniques like Shuffle-LAGAN (Brudno M., Bioinformatics 2003b, 19 Suppl 1:I54-I62) or Markov random fields.

The invention allows to obtain favorable phenotype features, but avoiding unfavorable phenotype features. By using the sequences of the invention this may be reduced to practice for essentially all plants of commercial interest by simply screening the genome of the plant or protein occurring in the plant for compliance of partial sequences thereof with the above sequences. A "hit" identifies a cytokinin-receptor to be suppressed or reduced according to the invention.

For example, enhanced root branching may be obtained without dwarf growth of other plant parts. The same applies in an analogous manner to the seed size. As a result, yield parameters, like the harvest index are considerably improved.

The target tissue is preferably selected from the group consisting of "root tissue, embryo tissue, endosperm tissue, and aleurone tissue". If the target tissue is root tissue and other tissue is not target tissue, a plant is obtained, which shows enhanced root branching but unaltered shoot growth. If the target tissue is embryo tissue, endosperm tissue or aleurone tissue, but other tissue is not target tissue, larger seeds are obtained at unaltered other properties of the plant. It is, however, also possible to combine both said subgroups of target tissue, provided that other tissue is not target tissue. This will result in a plant, wherein both, root branching and seed size are enhanced. This is most favorable since the increase in yield parameters is enhanced further.

Generally, the activity of the cytokinin-receptor may be suppressed or reduced in several ways. First, it is possible to reduce the activity by blocking the cytokinin-receptors themselves. This may be done by expressing tissue-specifically one compound or several compounds, which bind to cytokinin-receptors competitively with cytokinins. Second, it is possible to genetically modify specifically the target tissue in such a manner, that the cytokinin-receptors, as expressed, are non-functional, i.e. that cytokinin-receptor mutants are expressed tissue specifically. Third, it is possible to inhibit or reduce the translation of cytokinin-receptor RNA. Fourth, it is possible to inhibit or reduce the transcription of cytokinin-receptor DNA. The two latter alternatives are summarized as an inhibition or reduction of the cytokinin-receptor expression. Tissue specific expression of a compound inhibiting or reducing the cytokinin-receptor activity may be achieved in that the expression of the compound is controlled by a regulatory element, which is tissue specific, i.e. promotes expression only in the target tissue and not in tissue not being target tissue.

In a preferred embodiment, the expression of a cytokinin-receptor is reduced by tissue-specific transcription of foreign RNAi or antisense RNA being under the control of a tissue-specific regulatory element.

The transgenic plant of the invention is naturally not capable of reducing cytokinin-receptor expression tissue-specifically. A foreign DNA sequence is introduced by genetic engineering, which encodes for at least one nucleic acid or at least one protein or peptide being inhibitory for a cytokinin-receptor, wherein the foreign DNA sequence stands under the control of a tissue-specific regulatory element.

The transgenic plant of the invention produces seeds of enhanced size and/or produces roots with enhanced branching.

The invention comprises parts, cells, or seeds of a transgenic plant according to the invention as well as plants or propagating material thereof regenerated from a transgenic plant according to the invention.

The invention further relates to a process for making a transgenic plant according to the invention, wherein a vector comprising a DNA sequence, which encodes for at least one nucleic acid or at least one protein or peptide being inhibitory for a cytokinin-receptor, and which stands under the control of a tissue-specific regulatory element, is introduced in a gene technological manner into cells of a plant naturally not capable of reducing the cytokinin-receptor expression tissue-specifically, wherein the cells are transformed. Transformed cells are selected and transformed plants are regenerated from said cells.

A transgenic plant, wherein the activity of a cytokinin-receptor is reduced, in particular a transgenic plant according to the invention, may be used for producing seeds of enhanced size, wherein the transgenic plants are cultured under culturing conditions and the preferably mature seeds are harvested, or for producing a live root system with enhanced branching, wherein the transgenic plants are cultured under culturing conditions.

The invention further comprises a method for enhancing the seed size or the root branching of a plant, wherein the activity of a cytokinin-receptor is selectively suppressed or reduced in a specific target tissue of the plant, and wherein the activity of the cytokinin-receptor is essentially unaltered in tissue not being target tissue. The target tissue is preferably selected from the group consisting of "root tissue, embryo tissue, endosperm tissue, and aleurone tissue". The activity of a cytokinin-receptor may be reduced by inhibiting or reducing the expression of the cytokinin-receptor. The expression of the cytokinin-receptor may be reduced by tissue-specific expression of foreign RNAi or antisense RNA being under the control of a tissue-specific regulatory element. The tissue-specific regulatory element may be a promotor selected from the group consisting of the elements of the table 1 or any other promoter of the said specificity. Further details about applicable promoters and how to identify such promoters are obtainable from the following data sources. Gene expression data (strength and specifity) are given at https://www.genevestigator.ethz.ch. The publication describing this is by Zimmermann et al., Plant Physiol. 136, 2621-2632 (2004). Based on these data the expert skilled in the art can identify promoters with the needed specificity of expression. The promoters used in the examples for seed enhancement are: The *FWA* promoter being described in Kinoshita et al., Science 303, 521-523 (2004). The activity of the *FIS2* promoter is described in Luo et al., PNAS 97, 10637-10642 (2000). The promoters used in the examples for root enhancement are: The root-specific *pyk10* promoter being described by Nitz et al., Plant Science 161, 337-346 (2001).

**Table 1: Promotors usable in the invention.**

| **I: CELL-SPECIFIC,TISSUE-SPECIFIC, AND ORGAN-SPECIFIC PROMOTERS** | | |
|---|---|---|
| **GENE SOURCE** | **EXPRESSION PATTERN** | **REFERENCE** |
| α-amylase (*Amy32b*) | aleurone | Lanahan, M.B., et al., Plant Cell 4:203-211, 1992; Skriver, K., et al. Proc. Natl. Acad. Sci. (USA) 88: 7266-7270, 1991 |
| cathepsin β-like gene | aleurone | Cejudo, F.J., et al. Plant Molecular Biology 20:849-856, 1992. |
| *Agrobacterium rhizogenes rolB* | cambium | Nilsson et al., Physiol. Plant. 100:456-462, 1997 |
| AtPRP4 | flowers | http://salus.medium.edu/mmg/tiemey/ html |
| chalcone synthase (chsA) | flowers | Van der Meer, et al., Plant Mol. Biol. 15, 95-109, 1990. |
| LAT52 | anther | Twell et al Mol. Gen Genet. 217:240-245 (1989) |
| *apetala-3* | flowers | |
| chitinase | fruit (berries, grapes, etc) | Thomas *et al.* CSIRO Plant Industry, Urrbrae, South Australia, Australia; http://winetitles.com.au/gwrdc/csh95-1.html |
| rbcs-3A | green tissue (eg leaf) | Lam, E. et al., The Plant Cell 2: 857-866, 1990.; Tucker et al., Plant Physiol. 113: 1303-1308, 1992. |
| leaf-specific genes | leaf | Baszczynski, et al., Nucl. Acid Res. 16: 4732, 1988. |
| AtPRP4 | leaf | http://salus.medium.edu/mmg/tierney/ html |
| chlorella virus adenine metfiyltransferase gene promoter | leaf | Mitra and Higgins, 1994, Plant Molecular Biology 26: 85-93 |
| aldP gene promoter from rice rbcs promoter from rice or tomato | leaf | Kagaya et al., 1995, Molecular and General Genetics 248: 668-674 |
| | leaf | Kyozuka et al., 1993, Plant Physiology 102: 991-1000 |
| *Pinus cab-6* | leaf | Yamamoto et al., Plant Cell Physiol. 35:773-778. 1994. |
| rubisco promoter | leaf | |
| cab (chlorophyll a/b/binding protein | leaf | |
| SAM22 | senescent leaf | Crowell, et al., Plant Mol. Biol. 18: 459-466, 1992. |
| *Itp gens (lipid transfer gene)* | | Fleming, et al, Plant J. 2, 855-862*.* |
| *R. japonicum nif* gene | Nodule | United States Patent No. 4, 803, 165 |
| *B. japonicum nifH* gene | Nodule | United States Patent No. 5, 008, 194 |
| GmENOD40 | Nodule | Yang, et al., The Piant J. 3: 573-585. |
| PEP carboxylase (PEPC) | Nodule | Pathirana, et al., Plant Mol. Biol. 20: 437-450, 1992. |
| leghaemoglobin (Lb) | Nodule | Gordon, et al., J. Exp. Bot. 44: 1453-1465, 1993. |
| *Tungro bacilliform* virus gene | phloem | Bhattacharyya-Pakrasi, et al, The Plant J. 4: 71-79, 1992. |
| pollen-specific genes | pollen; microspore | Albani, et al., Plant Mol. Biol. 15: 605, 1990; Albani, et al., Plant Mol. Biol. 16: 501, 1991) |
| Zm13 | pollen | Guerrero et al Mol. Gen. Genet. 224:161-168 (1993) |
| apg gene | microspore | Twell et al Sex. Plant Reprod. 6:217-224(1993) |
| maize poilen-specific gene | pollen | Hamilton, et al., Plant Mol. Biol. 18: 211-218, 1992. |
| sunflower pollen- expressed gene | pollen | Baltz, et al., The Plant J. 2: 713-721, 1992. |
| *B. napus* pollen- specific gene | pollen;anther;tapetum | Amoldo, et al., J. Cell. Biochem., Abstract No. Y101, 204, 1992. |
| root-expressible genes | roots | Tingey, et al., EMBO J. 6: 1, 1987. |
| tobacco auxin-inducible gene | root tip | Van der Zaal, et al., Plant Mol. Biol. 16, 983, 1991. |
| β-tubulin | root | Oppenheimer, et al., Gene 63: 87, 1988. |
| tobacco root-specific genes | root | Conkling, et al., Plant Physiol. 93: 1203, 1990. |
| *B. napus* G1-3b gene | root | United States Patent No. 5, 401, 836 |
| SbPRPI | roots | Suzuki et al., Plant Mol. Biol. 21: 109-119, 1993. |
| AtPRP1; AtPRP3 | roots; root hairs | http://salus.medium.edu/mmg/tierney/ html |
| RD2 gene | root cortex | http://www2.cnsu.edu/ncsu/research |
| TobRB7 gene | root vasculature | http://www2.cnsu.edu/ncsu/research |
| AtPRP4 | leaves; flowers; lateral root primordia | http://salus.medium.edu/mmg/tierney/ html |
| seed-specific genes | seed | Simon, et al., Plant Mol. Biol. 5: 191, 1985; Scofield, et al., J. Biol. Chem. 262: 12202, 1987.; Baszczynski, et al., Plant Mol. Biol. 14: 633, 1990. |
| Brazil Nut albumin | seed | Pearson, et al., Plant Mol. Biol. 18: 235-245, 1992. |
| legumin | seed | Eiiis, et ai., Plant Mol. Biol. 10: 203-214, 1988. |
| glutelin (rice) | seed | Takaiwa, et al., Mol. Gen. Genet. 208: 15-22, 1986; Takaiwa, et al., FEBS Letts. 221: 43-47, 1987. |
| zein | seed | Matzke et al Plant Mol Biol, 14(3):323-321990 |
| napA | seed | Stalberg, et al, Planta 199: 515-519, 1996. |
| wheat LMW and HMW glutenin-1 | endosperm | Mol Gen Genet 216:81-90, 1989; NAR 17:461-2, 1989 |
| wheat SPA | seed | Albanl et al, Plant Cell, 9: 171-184, 1997 |
| wheat α, β, γ-gliadins | endosperm | EMBO 3:1409-15, 1984 |
| barley *Itr1* promoter | endosperm | |
| barley B1, C, D, hordein | endosperm | Theor Appl Gen 98:1253-62, 1999; Plant J 4:343-55, 1993; Mot Gen Genet 250:750-60. 1996 |
| barley DOF | endosperm | Mena et al, The Plant Journal, 116(1): 53-62, 1998 |
| *blz2* | endosperm | EP991 06056.7 |
| synthetic promoter | endosperm | Vicente-Carbajosa et al., Plant J. 13: 629-640, 1998. |
| rice prolamin NRP33 | endosperm | Wu et al, Plant Cell Physiology 39(8) 885-889, 1998 |
| rice α-globulin Glb-1 | endosperm | Wu et al, Plant Cell Physiology 39(8) 885-889, 1998 |
| rice OSH1 | embryo | Sato et al, Proc. Natl. Acad. Sci. USA, 93:8117-8122, 1996 |
| rice α-globulin REB/OHP-1 | endosperm | Nakase et al. Plant Mol. Biol. 33: 513-522, 1997 |
| rice ADP-gluoose PP | endosperm | Trans Res 6:157-68, 1997 |
| maize ESR gene family | endosperm | Plant J 12:235-46, 1997 |
| sorgum γ-kafirin | endosperm | PMB 32:1029-35, 1996 |
| KNOX | embryo | Postma-Haarsma et al, Plant Mol. Biol. 39:257-71, 1999 |
| rice oleosin | embryo and aleuron | Wu et at, J. Biochem., 123:386, 1998 |
| sunflower oleosin | seed (embryo and dry seed) | Cummins, et al., Plant Mol. Biol. 19: 873-876, 1992 |
| *LEAFY* | shoot meristem | Weigel et al., Cell 69:843-859, 1992. |
| *Arabidopsis thaliana knat1* | shoot meristem | Accession number AJ131822 |
| *Malus domestica kn1* | shoot meristem | Accession number Z71981 |
| *CLAVATA1* | shoot meristem | Accession number AF049870 |
| stigma-specific genes | stigma | Nasrallah, et al., Proc. Natl. Acad. Sci. USA 85: 5551, 1988; Trick, et al., Plant Mol. Biol. 15:203, 1990. |
| class I patatin gene | tuber | Liu et al., Plant Mol. Biol. 153:386-395, 1991. |
| PCNA rice | meristem | Kosugi et al, Nucleic Acids Research 19:1571-1576, 1991; Kosugi S. and Ohashi Y, Plant Cell 9:1607-1619, 1997. |
| Pea TubA1 tubulin | Dividing cells | Stotz and Long, Plant Mol.Biol. 41, 601-614. 1999 |
| *Arabidopsis* cdc2a | cycling cells | Chung and Parish, FEBS Lett, 3;362(2):215-9, 1995 |
| *Arabidopsis* Rop1A | Anthers; mature pollen + pollen tubes | Li et al. 1998 Plant Physiol 118, 407-417. |
| *Arabidopsis* AtDMC1 | Meiosis-associated | Klimyuk and Jones 1997 Plant J. 11, 1-14. |
| Pea PS-IAA4/5 and PS-IAA6 | Auxin-inducible | Wong et al. 1996 Plant J. 9, 587-599. |
| Pea farnesyltransterase | Meristematic tissues; phloem near growing tissues; light- and sugar-repressed | Zhou et al. 1997 Plant J. 12, 921-930 |
| Tobacco (*N. sylvestris*) cyclin B1;1 meristematic tissue | Dividing cells / | Trehin et al. 1997 Plant Mol. Biol. 35, 667-672. |
| *Catharanthus roseus* Mitotic cyclins CYS (A-type) and CYM (B-type) | Dividing cells / meristematic tissue | Ito et al. 1997 Plant J. 11, 983-992 |
| *Arabldopsis* cyc1 At (=cyc B1;1) and cyc3aAt (A-type) meristematic tissue | Dividing cells / | Shaul et al. 1996 Proc.Natl.Acad.Sci.U.S.A 93, 4868-4872. |
| *Arabidopsis* tef1 promoter box meristematic tissue | Dividing cells / | Regad et al. 1995 Mol.Gen.Genet. 248, 703-711. |
| *Catharanthus roseus* cyc07 meristematic tissue | Dividing cells / | Ito et al. 1994 Plant Mol.Biol. 24, 863-878. |

| **III: EXEMPLARY STRESS-INDUCIBLE PROMOTERS** | | |
|---|---|---|
| **NAME** | **STRESS** | **REFERENCE** |
| P5CS pyrroline-5-carboxylate (delta(1)-syntase) | salt, water | Zhang et al. Plant Science. 129: 81-89, 1997 |
| cor 15a | cold | Hajola et al., Plant Physiol 93: 1246-1252, 1990 |
| cor15b | cold | Wlihelm et al., Plant Mol Biol. 23:1073-1077, 1993 |
| cor15a (-305 to +78 nt) | cold, drought | Baker et al., Plant Mol Biol. 24:701-713, 1994 |
| rd29 | salt, drought, cold | Kasuga et al., Nature Biotechnology 18:287-291, 1999 |
| heat shock proteins, including artificial promoters containingthe heat shock element (HSE) | heat | Barros et al., Plant Mol Biol 19: 665-75, 1992. Marrs et al., Dev Genet.14: 27-41, 1993. Schoffl et al., Mol Gen Gent, 217:246-53,1989. |
| smHSP (small heat | heat shock proteins) | Waters et al, J Experimental Botany 47:325-338, 1996 |
| wcs120 | cold | Ouellet et al., FEBS Lett. 423: 324-328, 1998 |
| ci7 | cold | Kirch et al., Plant Mol Biol 33: 897-909, 1997 |
| Adh | cold, drought, hypoxia | Dolferus et al., Plant Physiol 105: 1075-87, 1994 |
| pwsl18 | water: salt and drought | Joshee et al., Plant Cell Physiol 39: 64-72, 1998 |
| ci21A | cold | Schneider et al., Plant Physiol 113: 335-45,1997 |
| Trg-31 | drought | Chaudhary et al., Plant Mol Biol 30: 1247-57, 1996 |
| osmotin | osmotic | Raghothama et al., Plant Mol Biol 23: 1117-28, 1993 |
| Rab17 | osmotic, ABA | Vilardell et al., Plant Mol Biol 17: 985-93, 1991 |
| lapA | wounding, enviromental | WO99/03977 University of California/INRA |

| **IV: EXEMPLARY PATHOGEN-INDUCIBLE PROMOTERS** | | |
|---|---|---|
| **NAME** | **PATHOGEN** | **REFERENCE** |
| RB7 | Root-knot nematodes (Meloidogyne spp.) | US5760386 - North Carolina State University; Opperman et al (1994) Science 263: 221-23. |
| PR-1, 2, 3, 4, 5, 8, 11 | fungal, viral, bacterial | Ward et al (1991) Plant Cell 3: 1085-1094; Reise et al 1996; Lebel et al (1998), Plant J, 16(2):223-33; Melchers et al (1994), Plant J, 5(4):469-80; Lawton et al (1992), Plant Mol Biol, 19(5):735-43. |
| HMG2 | nematodes | WO9503690 - Virginia Tech Intellectual Properties Inc. |
| Abi3 | Cyst nematodes (Heterodera spp.) | Unpublished |
| ARM1 | nematodes | Barthels et al., (1997) The Plant Cell 9, 2119-2134. WO 98/31822 - Plant Genetic Systems |
| Att0728 | nematodes | Barthels et al., (1997) The Plant Cell 9, 2119-2134. PCT/EP98/07761 |
| Att1712 | nematodes | Barthels et al., (1997) The Plant Cell 9, 2119-2134. PCT/EP98/07761 |
| Gst1 | Different types of pathogens | Strittmatter et al (1996) Mol. Plant-Microbe Interact. 9, 68-73. |
| *LEMMI* | nematodes | WO 92/21757*- Plant Genetic Systems* |
| CLE | geminivirus | PCT/EP99/03495 *- CINESTAV* |
| PDF1.2 | Fungal including *Alternaria brassicicola* and *Botrytis cinerea* | Manners et al (1998), Plant Mol Biol, 38(6):1071-80. |
| Thi2.1 | Fungal - *Fusarium oxysporum f sp. matthiolae* | Vignutelli et al (1998) Plant J;14(3):285-95 |
| DB#226 | nematodes | Bird and Wilson (1994) Mol. Plant-Microbe Interact., 7, 419-42 WO 95.322888 |
| DB#280 | nematodes | Bird and Wilson (1994) Mol. Plant-Microbe Interact., 7, 419-42 WO 95.322888 |
| Cat2 | nematodes | Niebel et al (1995) Mol Plant Microbe Interact 1995 May-Jun;8(3):371-8 |
| □Tub | nematodes | Aristizabal et al (1996), 8th International Congress on Plant-Microbe Interaction, Knoxville US B-29 |
| SHSP | nematodes | Fenoll et al (1997) In: Cellular and molecular aspects of plant-nematode interactions. Kluwer Academic, C. Fenoll, F.M.W. Grundler and S.A. Ohl (Eds.), |
| Tsw12 | nematodes | Fenoll et al (1997) In: Cellular and molecular aspects of plant-nematode interactions. Kluwer Academic, C. Fenoll, F.M.W. Grundler and S.A. Ohl (Eds.) |
| Hs1 (pro1) | nematodes | WO 98/122335 - Jung |
| NsLTP | viral, fungal, bacterial | Molina & Garc'ia-Olmedo (1993) FEBS Lett, 316(2):119-22 |
| RIP | viral, fungal | Tumer et al (1997) Proc Natl Acad Sci U S A, 94(8):3866-71 |

In a method of the invention, the plant is naturally not capable of reducing cytokinin-receptor expression tissue-specifically, wherein a foreign DNA sequence is introduced by genetic engineering, which encodes for at least one nucleic acid or at least one protein or peptide being inhibitory for cytokinin-receptor, and wherein the foreign DNA sequence stands under the control of a tissue-specific regulatory element. In this method the plant produces seeds of enhanced size and/or produces roots with enhanced branching. This may be done in the way that the plant is cultured under culturing conditions.

Reference herein to a "promoter" is to be taken in its broadest context and includes the transcriptional regulatory sequences derived from a classical eukaryotic genomic gene, including the TATA box which is required for accurate transcription initiation, with or without a CCAAT box sequence and additional regulatory or control elements (i.e. upstream activating sequences, enhancers and silencers) which alter gene expression in response to developmental and/or external stimuli, or in a tissue-specific manner. The term "promoter" also includes the transcriptional regulatory sequences of a classical prokaryotic gene, in which case it may include a -35 box sequence and/or a -10 box transcriptional regulatory sequences. The term "promote" is also used to describe a synthetic or fusion molecule, or derivative which confers, activates or enhances expression of a nucleic acid molecule in a cell, tissue or organ. Promoters may contain additional copies of one or more specific regulatory elements, to further enhance expression and/or to alter the spatial expression and/or temporal expression of a nucleic acid molecule to which it is operably connected. Such regulatory elements may be placed adjacent to a heterologous promoter sequence to drive expression of a nucleic acid molecule in response to e.g. copper, glucocorticoids, dexamethasone, tetracycline, gibberellin, cAMP, abscisic arid, auxin, wounding, ethylene, jasmonate or salicylic acid or to confer expression of a nucleic acid molecule to specific cells, tissues or organs such as meristems, leaves, roots, embryo, flowers, seeds or fruits. In the context of the present invention, the promoter preferably is a plant-expressible promoter sequence. Promoters that also function or solely function in non-plant cells such as bacteria, yeast cells, insect cells and animal cells are not excluded from the invention. By "plant-expressible" is meant that the promoter sequence, including any additional regulatory elements added thereto or contained therein, is at least capable of inducing, conferring, activating or enhancing expression in a plant cell, tissue or organ, preferably a monocotyledonous or dicotyledonous plant cell, tissue, or organ. The terms "plant-operable" and "operable in a plant" when used herein, in respect of a promoter sequence, shall be taken to be equivalent to a plant-expressible promoter sequence. Regulatable promoters as part of a binary viral plant expression system are also known to the skilled artisen (Yadav 1999 - W09922003; Yadav 2000 - W00017365). In the present context, a "regulatable promoter sequence" is a promoter that is capable of conferring expression an a structural gene in a particular cell, tissue, or organ or group of cells, tissues or organs of a plant, optionally under specific conditions, however does generally not confer expression throughout the plant under all conditions. Accordingly, a regulatable promoter sequence may be a promoter sequence that confers expression an a gene to which it is operably connected in a particular location within the plant or alternatively, throughout the plant under a specific set of conditions, such as following induction of gene expression by a chemical compound or other elicitor. Preferably, the regulatable promoter used in the performance of the present invention confers expression in a specific location within the plant, either constitutively or following induction, however not in the whole plant under any circumstances. Included within the scope of such promoters are cell-specific promoter sequences, tissue-specific promoter sequences, organ-specific promoter sequences, cell cycle specific gene promoter sequences, inducible promoter sequences and constitutive promoter sequences that have been modified to confer expression in a particular part of the plant at any one time, such as by integration of said constitutive promoter within a transposable genetic element (Ac, Ds, Spm, En, or other transposon). Similarly, the term "tissue-specific" shall be taken to indicate that expression is predominantly in a particular tissue or tissue-type, preferably of plant origin, albeit not necessarily exclusively in said tissue or tissue-type. Similarly, the term "organ-specific" shall be taken to indicate that expression is predominantly in a particular organ, preferably of plant origin, albeit not necessarily exclusively in said organ. Similarly, the term "cell cycle specific" shall be taken to indicate that expression is predominantly cyclic and occurring in one or more, not necessarily consecutive phases of the cell cycle albeit not necessarily exclusively in cycling cells, preferably of plant origin. Those skilled in the art will be aware that an "inducible promoter" is a promoter the transcriptional activity of which is increased or induced in response to a developmental, chemical, environmental, or physical stimulus. Similarly, the skilled craftsman will understand that a "constitutive promoter" is a promoter that is transcriptionally active throughout most, but not necessarily all parts of an organism, preferably a plant, during most, but not necessarily all phases of its growth and development. Those skilled in the art will readily be capable of selecting appropriate promoter sequences for use in regulating appropriate expression of the receptor inhibitory protein or RNAi or antisense nucleic acid from publicly-available or readily-available sources, without undue experimentation. Placing a nucleic acid molecule under the regulatory control of a promoter sequence, or in operable connection with a promoter sequence, means positioning said nucleic said molecule such that expression is controlled by the promoter sequence. A promoter is usually, but not necessarily, positioned upstream, or at the 5'-end, and within 2 kb of the start site of transcription, of the nucleic acid molecule which it regulates. In the construction of heterologous promoter/structural gene combinations it is generally preferred to position the promoter at a distance from the gene transcription start site that is approximately the same as the distance between that promoter and the gene it controls in its natural setting (i.e., the gene from which the promoter is derived). As is known in the art, some variation in this distance can be accommodated without loss of promoter function. Similarly, the preferred positioning of a regulatory sequence element with respect to a heterologous gene to be placed under its control is defined by the positioning of the element in its natural setting (i.e., the gene from which it is derived). Again, as is known in the art, some variation in this distance can also occur. Examples of promoters suitable for use in gene constructs of the present Invention include those listed in Table 1, amongst others. The promoters listed in Table 1 are provided for the purposes of exemplification only and the present invention is not to be linked by the list provided therein. Those skilled in the art will readily be in a position to provide additional promoters that are useful in performing the present invention. In the case of constitutive promoters or promoters that induce expression throughout the entire plant, it is preferred that such sequences are modified by the addition of nucleotide sequences derived from one or more of the tissue-specific promoters listed in Table 1, or alternatively, nucleotide sequences derived from one or more of the above-mentioned tissue-specific inducible promoters, to confer tissue-specificity thereon. For example, the CaMV 35S promoter may be modified by the addition of maize Adh1 promoter sequence, to confer anaerobically-regulated root-specific expression thereon, as described previously (Ellis et al., 1987). Another example describes conferring root specific or root abundant gene expression by fusing the CaMV35S promoter to elements of the maize glycine-rich protein GRP3 gene (Feix and Wulff 2000 - W00015662). Such modifications can be achieved by routine experimentation by those skilled in the art. The term "terminator" refers to a DNA sequence at the end of a transcriptional unit which signals termination of transcription. Terminators are 3'-non-translated DNA sequences containing a polyadenylation signal, which facilitates the addition of polyadenylate sequences to the 3'-end of a primary transcript. Terminators active in cells derived from viruses, yeasts, moulds, bacteria, insects, birds, mammals and plants are known and described in the literature. They may be isolated from bacteria, fungi, viruses, animals and/or plants. Examples of terminators particularly suitable for use in the gene constructs of the present invention include the Agrobacterium tumefaclens nopaline synthase (NOS) gene terminator, the Agrobacterium tumefaciens octopine synthase (OCS) gene terminator sequence, the Cauliflower mosaic virus (CaMV) 35S gene terminator sequence, the Oryza sativa ADP-glucose pyrophosphorylase terminator sequence (t3'Bt2), the Zea mays zein gene terminator sequence, the rbcs-1A gene terminator, and the rbcs-3A gene terminator sequences, amongst others. Preferred promoter sequences of the invention include root specific promoters such as but not limited to the ones listed in Table 1 and as outlined in the Examples. Those skilled in the art will be aware of additional promoter sequences and terminator sequences which may be suitable for use in performing the invention. Such sequences may readily be used without any undue experimentation.

The invention further comprises a method for making seeds of enhanced size, wherein the transgenic plant, parts thereof, or seeds according to the invention are cultured under culturing conditions and preferably mature seeds being produced thereby are harvested, as well as seeds obtainable by a method of any of the claims 14 to 22.

Plants with an enhanced root system are better adapted to stress, they enhance plant vigour, they grow better on soil poor in nutritional elements (minerals), they show improved growth with limited water resources and enhanced resistance to drought finally leading to improved yield parameters, in particular an improved harvest index. They can also be used for phytoremediation, i.e. plant mediated removal of toxic substances from soil, and/or prevention and/or arrest of soil erosion. The methods of the invention as well as the plants thereof result in an only slightly enhanced growth of the primary root but strongly enhanced root branching. This leads after 10 to 30 days of culturing to a two- to fourfold enhanced root system (defined as the dry weight of the roots compared to the dry weight of the roots of the wild type plant cultured under the same conditions).

Improvement of the root system in particular is favorable for staple crops, like sugar beet, manioc, yams, sweet potatoe, vegetables with consumable root parts like carrots and radish, and medicinal plants with usable root parts, like ginseng. But also the yield parameters of other crops like wheat, maize etc. are increased, since the plant growth is improved due to the comparatively better uptake of water and nutritional substances from the soil.

Plant with an increased seed size provide a higher yield parameters for obvious reasons.

The present invention is applicable to any plant, in particular to monocotyledonous plants and dicotyledonous plants including a fodder or forage legume, ornamental plant, food crop, tree, or shrub selected from the list comprising Acada spp., Acer spp., Actinidia spp.,Aesculus spp., Agathis australis, Albizia amara, Alsophila tricolor, Andropogon spp., Arachis spp, Areca catechu, Astelia fragrans, Astragatus cicer, Baikiaea pludjuga, Betula spp., Brasslca spp., Bruguiera gymnorrhiza, Burkea africana, Butea frondosa, Cadaba farmosa, Calilandra spp, Camellia sinensts, Canna Indica, Capsicum spp., Cassia spp., Centroema pubescens, Chaenomeles spp.,Cinnamomum cassia, Coffea arablca, Colophospermum mopane, Coronillia varia, Cotoneaster serotina, Crataegus spp., Cucumis spp., Cupressus spp., Cyathea dealbata, Cydonia oblonga, Cryptomeda japonica, Cymbopogon spp., Cynthea dealbata, Cydonia oblonga, Dalbergia monetaria, Davallia divaricata, Desmodium spp., Dlcksonia squarosa, Diheteropogon amplectens, Dioclea spp, Dolichos spp., Dorycnium rectum, Echinochloa pyramidatts, Ehrartfa spp., Eleusine coracana, Eragrestis spp., Erythrina spp., Eucalyptus spp., Euclea schimperi, Eulalla villosa, Fagopyrum spp., Feijoa sellowfana, Fragaria spp., Flemingta spp, Freycinetia banksli, Geranium thunbergii, Ginkgo biloba, Glycine javanica, Glidcidia spp, Gossypium hirsutum, Grevillea spp., Gufbourtia coleosperma, Hedysanrm spp., Hemarthia altissima, Heteropogon contortus, Hordeum vulgare, Hyparrhenta rufa, Hypericum erectum, Hyperthelfa dissoluta, Indigo incamata, Iris spp., Leptarrhena pyrolifolia, Lespediza spp., Lettuca spp., Leucaena leucocephala, Lcudetia simplex, Lotonus bainesit, Lotus spp., Macrotyloma exillare, Malus spp., Manihot esculenta, Medicago sativa, Metasequoia glyptostroboides, Muse sapientum, Nicotianum spp., Onobrychis spp., Ornithopus spp., Oryza spp., Peltophorum afncanum, Penntsetum spp., Persea gratissima, Petunia spp., Phaseolus spp., Phoenix canariensis, Phormium cookfanum, Photinia spp., Picea glauca, Pinus spp., Pisum sativum, Podocarpus totara, Pogonarthria flecltii, Pogonarthria squarrosa, Populus spp., Prosopis cfneraria, Pseudotsuga menziesli, Pterolobfum stellatum, Pyrus communis, Quercus spp., Rhaphiolepsis umbellata, Rhopalostylis sapida, Rhus natalensis, Ribes grossularia, Ribes spp., Robinla pseudoacacia, Rosa spp., Rubus spp., Salix spp., Schyzachyrium sanguineum, Sciadopitys verticillata, Sequoia sempervfrens, Sequoiadendron giganteum, Sorghum bicolor, Spinacia spp., Sporobolus flmbriatus, Siiburus alopecurotdes, Stylosanthos humtlis, Tadehagi spp, Taxodium distichum, Themeda triandra, Trffolium spp., Triticum spp., Tsuga heterophylia, Vaccinium spp., Vicia spp.Vitis vinffera, Watsonia pyramidata, Zantedcischla aethiopica, Zea mays, amaranth, artichoke, asparagus, broccoli, brussel sprout, cabbage, canola, carrot, cauliflower, celery, collard greens, flax, kale, lentil, oilseed rape, okra, onion, potato, rice, soybean, straw, sugarbeet, sugar cane, sunflower, tomato, squash, and tea, amongst others.

Means for introducing foreign resp. recombinant DNA into plant tissue or cells include, but are not limited to, transformation using CaCl₂ and variations thereof, in particular the method described by Hanahan (1983), direct DNA uptake into protoplasts (Krens et al, 1982; Paszkowski et al, 1984), PEG-mediated uptake to protoplasts (Armstrang et al, 1990) microparticle bombardment, electroporation (Fromm et al., 1985), microinjection of DNA (Crossway et al., 1986), microparticle bombardment of tissue explants or cells (Christau et al, 1988; Sanford, 1988), vacuum-infiltration of tissue with nucleic acid, or in the case of plants, T-DNA-mediated transfer from Agrobacterium to the plant tissue as described essentially by An et al.(1985), Dodds et al., (1985), Herrera-Estrella et al. (1983a, 1983b, 1985). Methods for transformation of monocotyledonous plants are well known in the art and include Agrobacterium-mediated transformation (W09748814; W09854961; W09400977; W09817813; W09904618; W09506722), microprojectile bombardment (US5969213; US5736369; W09413822; US5874265/US5990390; US5405765; US5955362), DNA uptake (W09318168), microinjection of Agrobacterium cells (DE4309203) and sonication (US5693512). For microparticle bombardment of cells, a microparticle is propelled into a cell to produce a transformed cell. Any suitable ballistic cell transformation methodology and apparatus can be used in performing the present Invention. Exemplary apparatus and procedures are disclosed in US 5,122,466 and US 4,945,050. When using ballistic transformation procedures, the gene construct may incorporate a plasmid capable of replicating in the cell to be transformed. Examples of microparticles suitable for use in such systems include 1 to 5 µm gold spheres. The DNA construct may be deposited an the microparticle by any suitable technique, such as by precipitation. A whole plant may be regenerated from the transformed or transormed cell, in accordance with procedures well known in the art. Plant tissue capable of subsequent clonal propagation, whether by organogenesis or embryogenesis, may be transformed with a gene construct of the present invention and a whole plant regenerated therefrom. The particular tissue chosen will vary depending an the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The term "organogenesis", as used herein, means a process by which shoots and roots are developed sequentially from meristematic centres. The term "embryogenesis", as used herein, means a process by which shoots and roots develop together in a concerted fashion (not sequentially), whether from somatic cells or gametes. Preferably, the plant is produced according to the inventive method is transfected or transformed with a genetic sequence, or amenable to the introduction of a receptor inhibiting protein or receptor inhibitory nucleic acid, by any art-recognized means, such es microprojectile bombardment, microinjection, Agrobacterium-mediated transformation (including in planta transformation), protoplast fusion, or electroporation, amongst others. Most preferably said plant is produced by Agrobacterium-mediated transformation. Agrobacterium-mediated transformation or agrolistic transformation of plants, yeast, moulds or filamentous fungi is based an the transfer of part of the transformation vector sequences, called the T-DNA, to the nucleus and an integration of seid T-DNA in the genome of seid eukaryote. With "Agrobacterium" is meant a member of the Agrobacteriaceae, more preferably Agrobacterium or Rhizobacterium and most preferably Agrobacterium tumefaciens. With "T-DNA", or transferred DNA, is meant that part of the transformation vector flanked by T-DNA borders which is, after activation of the Agrobacterium virgenes, nicked at the T-DNA borders and is transferred as a single stranded DNA to the nucleus of an eukaryotic cell. When used herein, with "T-DNA borders", "T-DNA border region", or "border region" are meant either right T-DNA border (RB) or left T-DNA border (LB). Such a border comprises a core sequence flanked by a border inner region as part of the T-DNA flanking the border and/or a border outer region as part of the vector backbone flanking the border. The core sequences comprise 22 bp in case of octopine-type vectors and 25 bp in case of nopaline-type vectors. The core sequences in the right border region and left border region form imperfect repeats. Border core sequences are indispensable for recognition and processing by the Agrobacterium nicking complex consisting of at least VirD1 and VirD2. Core sequences flanking a T-DNA are sufficient to promote transfer of said T-DNA. However, efficiency of transformation using transformation vectors carrying said T-DNA solely flanked by said core sequences is low. Border inner and outer regions are known to modulate efficiency of T-DNA transfer (Wang et el. 1987). One element enhancing T-DNA transfer has been characterized and resides in the right border outer region and is called overdrive (Peralta et al. 1986, van Haaren et al. 1987). With "T-DNA transformation vector" or "T-DNA vector" is meant any vector encompassing a T-DNA sequence flanked by a right and left T-DNA border consisting of at least the right and left border core sequences, respectively, and used for transformation of any eukaryotic cell. With "T-DNA vector backbone sequence" or "T-DNA vector backbone sequences" is meant all DNA of a T-DNA containing vector that lies outside of the T-DNA borders and, more specifically, outside the nicking sites of the border core imperfect repeats. The current invention includes optimized T-DNA vectors such that vector backbone integration in the genome of a eukaryotic cell is minimized or absent. With "optimized T-DNA vector" is meant a T-DNA vector designed either to decrease or abolish transfer of vector backbone sequences to the genome of a eukaryotic cell. Such T-DNA vectors are known to the one familiar with the art and include those described by Hanson et al. (1999) and in W09901563. The current invention clearly considers the inclusion of a DNA sequence encoding an receptor inhibitory protein and/or inhibitory nucleic acid, like RNAi or antisense, in any T-DNA vector comprising binary transformation vectors, super-binary transformation vectors, co-integrate transformation vectors, bi-derived transformation vectors as well as in T-DNA carrying vectors used in agrolistic transformation. With "binary transformation vector" is meant a T-DNA transformation vector comprising: (a) a T-DNA region comprising at least one gene of interest and/or at least one selectable marker active in the eukaryotic cell to be transformed; and (b) a vector backbone region comprising at least origins of replication active in E. coli and Agrobacterium and markers for selection in E. coli and Agrobacterium. The T-DNA borders of a binary transformation vector can be derived from octopine-type or nopaline-type Ti plasmids or from both. The T-DNA of a binary vector is only transferred to a eukaryotic cell in conjunction with a helper plasmid: With "helper plasmid" is meant a plasmid that is stably maintained in Agrobacterium and is at least carrying the set of vir genes necessary for enabling transfer of the T-DNA. Said set of vir genes can be derived from either octopine-type or nopaline-type Ti plasmids or from both. With "super-binary transformation vector" is meant a binary transformation vector additionally carrying in the vector backbone region a vir region of the Ti plasmid pT1Bo542 of the super-virulent A. tumefaclens strain A281 (EP0604662, EP0687730). Super-binary transformation vectors arg used in conjunction with a helper plasmid. With "co-integrate transformation vector" is meant a T-DNA vector at least comprising: (a) a T-DNA region comprising at least one gene of interest and/or at least one selectable marker active in plants; and (b) a vector backbone region comprising at least origins of replication active in Escherichia coli and Agrobacterium, and markers for selection in E. coli and Agrobacterium, and a set of vir genes necessary for enabling transfer of the T-DNA. The T-DNA borders and said set of vir genes of a said T-DNA vector can be derived from either octopine-type or nopaline-type Ti plasmids or from both. With "Ri-derived plant transformation vector" is meant a binary transformation vector in which the T-DNA borders are derived from a Ti plasmid and said binary transformation vector being used in conjunction with a helper Ri-plasmid carrying the necessary set of vir genes. As used herein, the term "selectable marker gene" or "selectable marker" or "marker for selection" includes any gene which confers a phenotype an a cell in which it is expressed to facilitate the identification and/or selection of cells which are transfected or transformed with a gene construct of the invention or a derivative thereof. Suitable selectable marker genes contemplated herein include the ampicillin resistance (Amp^{r}, tetracycline resistance gene (Tc^{r}, bacterial kanamycin resistance gene (Kan^{r}), phosphinothricin resistance gene, neomycin phosphotransferase gene (nptlI), hygromycin resistance gene, β-glucuronidase (GUS) gene, chloramphenicol acetyltransferase (CAT) gene, green fluorescent protein (gfp) gene, and luciferase gene, amongst others. With "agrolistics"; "agrolistic transformation" or "agrolistic transfer" is meant here a transformation method combining features of Agrobacterium-mediated transformation and of biolistic DNA delivery. As such, a T-DNA containing target plasmid is co-delivered with DNA/RNA enabling in plantal production of VirD1 and VirD2 with or without, VirE2 (W09712046). With "foreign DNA" is meant any DNA sequence that is introduced in the host's genome by recombinant techniques. Said foreign DNA includes e.g. a T-DNA sequence or a part thereof such as the T-DNA sequence comprising the selectable marker in an expressible format. Foreign DNA furthermore include intervening DNA sequences as defined supra.

In the following the invention is explained in more detail by non-limiting examples.

### Example 1: novel AHK2/AHK3 double mutants and AHK2/AHK3/AHK4 triple mutants

The instant invention in addition comprises novel AHK2/AHK3 double mutants and AHK2/AHK3/AHK4 triple mutants, which are alleles of the mutants known from the state of the art. The novel mutants are characterized by the sequence information provided in the following.

### 1.1: Isolation of Novel T-DNA Insertion Alleles in the AHK2 and AHK3 Genes

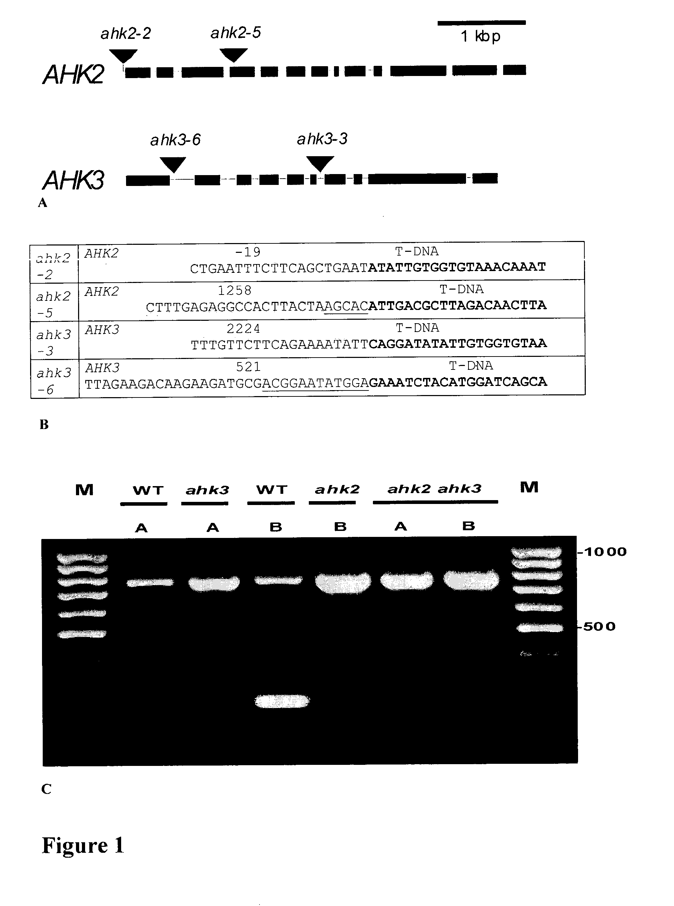

*Arabidopsis* lines carrying T-DNA insertions in the *AHK2* (At5g35750) and *AHK3* (At1g27320) genes were identified in different mutant collections. Two independent mutant alleles were isolated for each gene (Fig. 1A). All mutations are in the *Arabidopsis* Col-0 background. The exact T-DNA insertion sites were determined by sequencing (Fig. 1B) and a PCR-based strategy to identify heterozygote and homozygote insertional mutants was established for each mutant allele. One insertion in the *AHK2* gene was found to be in the fourth exon (Fig. 1B). This is a novel allele, which was named *ahk2-5,* as four other *ahk2* insertion mutants were described previously. The second *ahk2* mutant allele is identical to the known *ahk2-2,* it carries an insertion 19 bp upstream of the predicted translational start codon (Fig. 1B). *ahk3-6* is a novel insertion allele of the *AHK3* gene and carries an insertion at the 5' end of intron 1. The second isolated *ahk3* mutant allele is identical to the known *ahk3-3,* it has an insertion in the sixth intron (Figs. 1A,B). Fig. 1C shows that gene-specific transcripts were detected in wild-type but no gene-specific transcripts were detected in the *ahk2-5* and *ahk3-6* single mutants or in the *ahk2-5 ahk3-6* double mutant. It is therefore concluded that both mutations present novel null alleles. *ahk2-5, ahk3-6* and *cre1-2,* which carries an insertion in the first exon (Inoue et al., 2001), were used for experiments with single knockouts and all reported receptor mutant combinations. In the text the designations *ahk2, ahk3* and *cre1* are used for these alleles. Experiments reporting results for the *ahk2 ahk3* double mutant combination were carried out using both the *ahk2-5 ahk3-6* and *ahk2-2 ahk3-3* combinations. In all cases similar results were obtained with both mutant combinations.

### 1.2: ahk2 and ahk3 Single and Double Mutants Show Enhanced Cytokinin Resistance

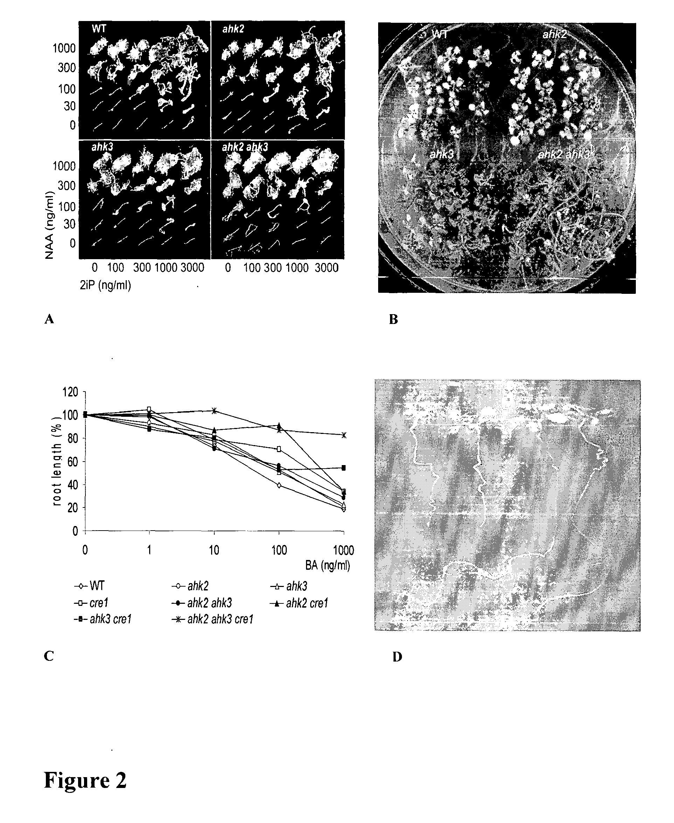

Several assays were used to test the cytokinin sensitivity of *ahk* mutants, in particular that of the novel *ahk2-5* and *ahk3-*6 alleles and their combination. Hypocotyl explants of ten day old seedlings were placed on media with different concentrations of auxin and cytokinin. Callus formation, i.e. dedifferentiation and growth of the tissue, and organ formation, i.e. induction of roots or shoots, was scored after four weeks. Fig. 2A shows that *ahk2* mutant explants displayed a reaction which was comparable to wild type. In contrast, explants of *ahk3* mutants had a significantly reduced ability to respond to cytokinin by callus or shoot formation. Explants of the double mutant *ahk2 ahk3* showed further enhancement of this phenotype (Fig. 2A). Three- to tenfold higher cytokinin concentrations than in wild type were required to induce callus formation and growth. Shoot formation was observed very rarely in *ahk2 ahk3* explants. Instead, they showed a tendency to differentiate into roots on low cytokinin concentrations while wild type did not grow on this medium (Fig. 2A). A similar result was obtained when the ability of plants to react to cytokinin was tested in vitro. Elevated levels of cytokinin inhibit wild type shoot growth, reduce the accumulation of leaf chlorophyll and suppress flower induction (Fig. 2B). *ahk2* mutants showed a similar inhibition reaction as wild type, while *ahk3* mutant seedlings grew better and formed darker green leaves. *ahk2 ahk3* double mutants showed a further degree of cytokinin resistance in this assay and flowered *in vitro* four weeks after sowing on medium supplemented with 40 ng/l BA (Fig. 2B). Cytokinin sensitivity of roots was tested *in vitro* on media with increasing cytokinin concentrations. All single mutants and mutant combinations were included in this test to study the relative contribution of all three receptors. Root growth of *cre1* and combinations of *cre1* with other *ahk* mutants showed increased cytokinin resistance (Fig. 2C). This confirms a main role of CRE1/AHK4 in sensing of exogenous cytokinin in the primary root meristem, with some contribution from AHK2 and AHK3 (Inoue et al., 2001; Higuchi et al., 2004; Nishimura et al., 2004). Functioning of AHK2 and AHK3 in the root independent of CRE1/AHK4 became apparent after prolonged exposure to cytokinin. Fig. 2D shows that roots of *ahk2 ahk3* double mutants were able to continue to grow slowly on medium containing 100 ng/l BA while wild type roots ceased growing.

### 1.3: Shoot Phenotype of Cytokinin Receptor Mutants

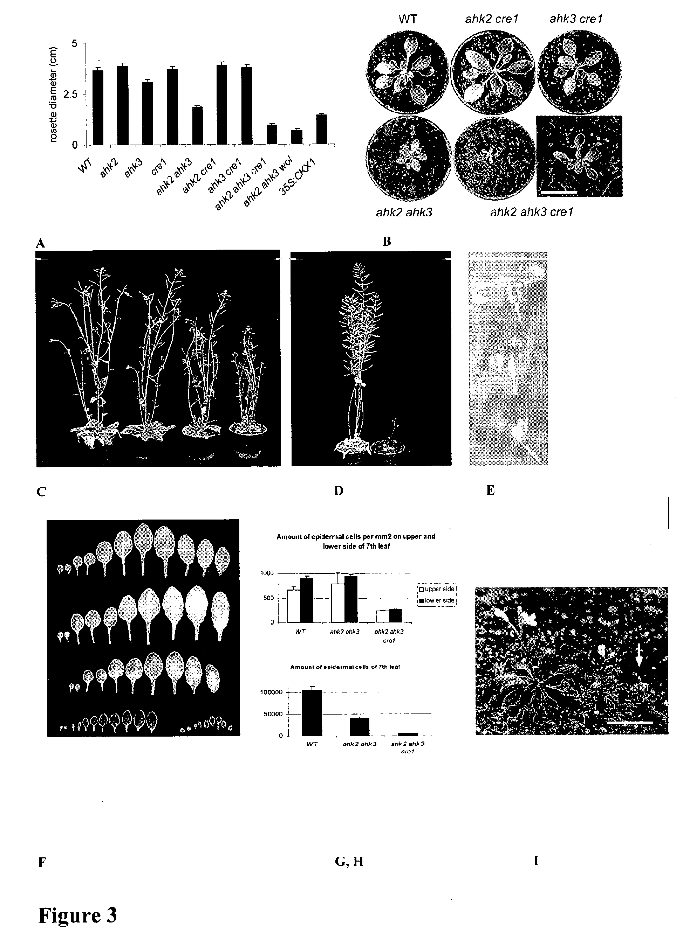

The development of single, double and triple mutant shoots was compared. Shoot development of *ahk2* and *cre1* single mutants was indistinguishable from wild type (Fig. 3 and data not shown). In contrast, the rosette diameter of *ahk3* mutants was reduced approximately 15% compared to wild type (Fig. 3A). Introgression of an *ahk2* mutant allele reduced rosette diameter further to about half the size of the wild type rosette (Figs. 3A,B). The rate of leaf formation (plastochrone) was not altered in *ahk2 ahk3* double mutants and flowering was induced at the same time as in wild type (data not shown). The final height of mutant plants was about two thirds of wild-type (Fig. 3C). Triple mutant plants showed an even greater reduction of shoot development, resulting in miniature plants (Fig. 3D) revealing that CRE1/AHK4 also functions in the shoot. Cotyledons of triple mutant seedlings had a reduced size (Fig. 3E) consistent with the role of cytokinin in promoting cotyledon expansion. Cotyledons were bent downwards indicating differential growth in the adaxial and abaxial sides (Fig. 3E). 25 DAG wild type plants had developed 14-16 leaves and started to flower. At that time triple mutant plants had only 7-8 leaves indicating a longer plastochrone. Flower induction was variable. On average, triple mutant plants flowered two to three weeks later than wild type. Triple mutant plants were almost completely infertile. However, in contrast to complete sterility reported by others (Nishimura et al., 2004; Higuchi et al., 2004) they self-fertilized and formed a few seeds under favorable temperature and light conditions. This suggests that infertility of triple mutants is a conditional phenotype. Leaves of *ahk2 ahk3* double mutants had a reduced length and width but the overall form and heteroblasty were not altered (Fig. 3F). Leaves of triple mutants were even smaller but had an unaltered length-to-width ratio (Fig. 3F and data not shown). Microscopic inspection of epidermal cells at different sites of the fifth leaf at maturity revealed that the average cell size on the upper and lower epidermis of *ahk2 ahk3* mutant leaves was about double the size of wild type cells (data not shown). Interestingly, cell size became similar to wild type in leaves that were formed at later stages (Fig. 3G; see also Nishimura et al., 2004). Leaf cell size of triple mutants was increased about threefold in older leaves (Fig. 3G) and in total less leaf cells were formed compared to wild type (Fig. 3H). An increase in cell size to compensate for the reduced number of cells has been reported for different cell cycle mutants and appears to be transient in the *ahk2 ahk3* double mutant but persistent in the triple mutant.

### 1.4: Senescence Retardation by Cytokinin in the Receptor Mutants

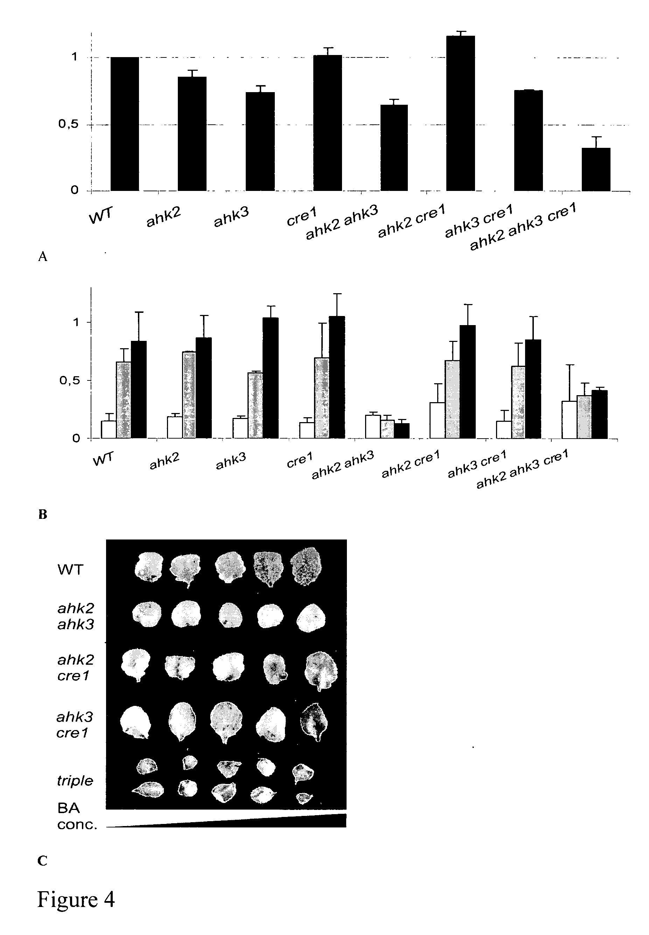

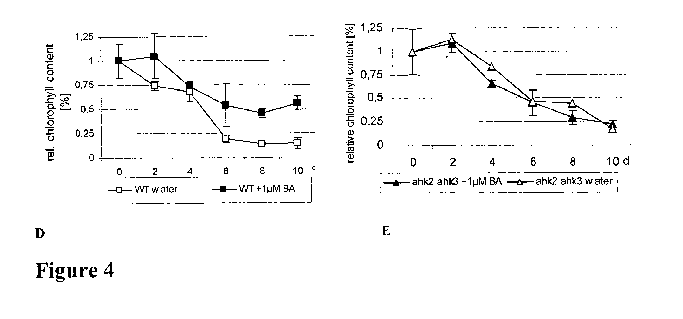

The influence of cytokinin on the chlorophyll content of leaves and their ability to retard leaf senescence was described soon after their discovery. The participation of the different receptors in mediating senescence retardation by cytokinin using dark-induced detached leaves was investigated. Fig. 4A shows that specific cytokinin receptor mutations cause a reduction of the leaf chlorophyll content. *ahk3* reduced the chlorophyll content to ca. 70% of wild type, *ahk2* had a weak and *cre1* no effect. Combination of *ahk3* with *ahk2* reduced the chlorophyll content further. A role of CRE1/AHK4 in assuring chlorophyll formation only became apparent in the triple mutant, which has ca. 35% of the chlorophyll of wild type (Fig. 4A). The contribution of each receptor in mediating cytokinin-dependent chlorophyll retention was tested. Fig. 4B shows that loss of a single receptor did not strongly alter the cytokinin responsiveness. A functional AHK3 receptor alone was sufficient to fully respond to exogenous cytokinin. However, the *ahk2 ahk3* double mutant had completely lost the ability to retain chlorophyll in response to cytokinin (Figs. 4B, C). Analysis of the kinetics of chlorophyll loss revealed that cytokinin slowed the loss of chlorophyll in wild type where its content stabilized after six days at around 60% of the original content (Fig. 4D). In contrast, the kinetics of chlorophyll loss was similar in *ahk2 ahk3* mutants in the absence and in the presence of cytokinin (Fig. 4E). These data reveal a major contribution of AHK3 in mediating cytokinin-dependent chlorophyll retention in leaves.

### 1.5: Seed Phenotype of Cytokinin Receptor Mutants

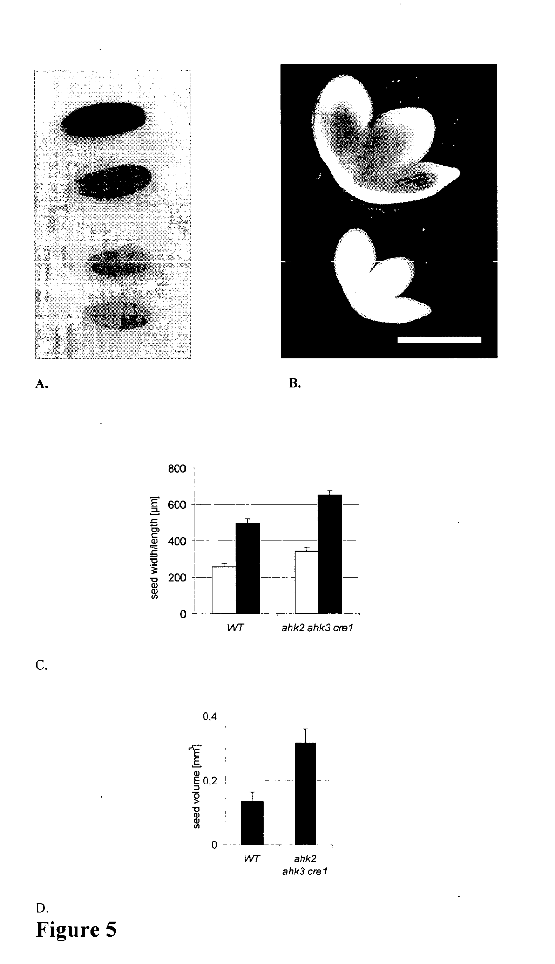

Seed size of single and double mutants was unchanged and microscopic inspection of embryos did not indicate gross developmental differences between mutant and wild type embryos (data not shown). However, seeds of the triple mutant were significantly increased in size (Fig. 5A). The increase of seed size was mainly due to an increased size of the embryos (Fig. 5B). Because of the low number of seeds seed mass was not measured but seed size instead. The seeds of the wild type and the triple mutant were both uniform in size. The average seed length and width of triple mutant seeds increased approximately 30% (Fig. 5C). Calculation of the seed volume by a spheroid formula revealed that the size of triple mutant seeds was increased by ca. 250% (Fig. 5D). It could be that the increase in seed size is an autonomous function of the embryo proper or a function linked to the genotype of the triploid endosperm, which results from fertilization of the central cells of the ovule, and/or of the maternal tissue, which includes the seed coat. In order to investigate this further we analyzed progeny seeds of reciprocal crosses between wild type and the triple mutant. Seeds formed on wild type plants had a normal size, while seeds formed by cross-pollinated triple mutants were increased in size. This indicated that the size increase is not or not solely a function of the embryo proper, which is heterozygote for all receptor mutant alleles in both progenies. Analysis of seeds of a plant that was homozygote for *ahk2 cre1* and heterozygote for the *ahk3* mutation showed a heterogenous size of the seeds among the progeny of this plant, in contrast to progenies obtained by self-fertilization of wild type or *ahk2 cre1* homozygote mutant lines, which form uniform seeds of similar size. Progeny of the said cross segregated 1:3 for the triple mutant phenotype. We selected microscopically among 60 randomly chosen seeds the 20 largest and the 20 smallest seeds and screened their phenotype after germination. If the large seed phenotype was linked at least partially to the genotype of the embryo one would expect an increased proportion of triple mutant phenotype among the larger seeds. 4 of the 16 selected larger seeds and 4 of the 16 selected smaller seeds showed the triple mutant phenotype. The 1:3 segregation ratios indicate that seed size does not depend on the embryonic genotype. If the embryo size was controlled maternally, uniform seeds of wild type size would be expected to form. The fact that seeds of significantly different size were found among the progeny indicates a role for the genotype of the endosperm. Self-pollination of a parental plant which is homozygote for *ahk2 cre1* and heterozygote for *ahk3* leads to triploid endosperm carrying four *ahk2 cre1* alleles and between one and three *ahk3* mutant alleles. Therefore different allele combinations in different endosperms could be the reason for the variability in seed size.

### 1.6: Germination Phenotype of Cytokinin Receptor Mutant Seeds

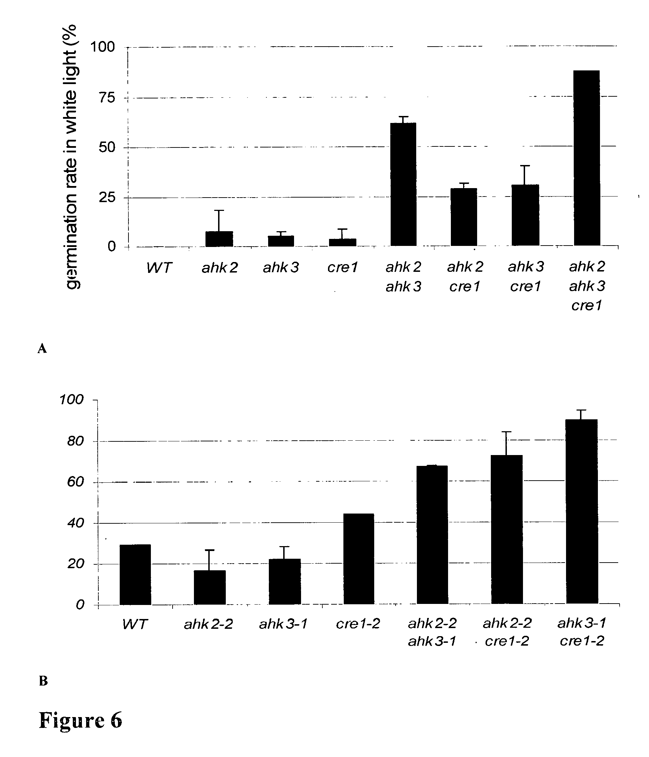

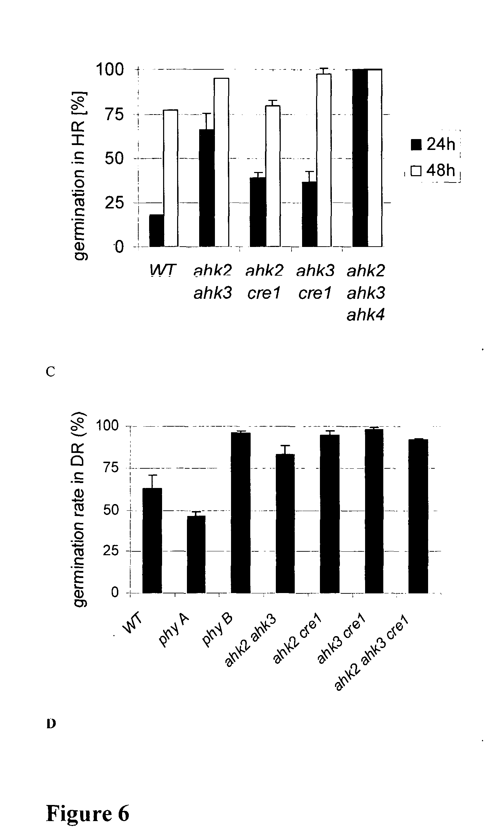

The timing of germination was different in the receptor mutants. After sowing, all seeds were pretreated for 2 days at 4 °C in the dark. Wild type seeds started to germinate between 12 h and 24 h after transfer to light and ambient temperature. 30 h after sowing 50% of wild type seeds had germinated (Fig. 6A). Seeds of the *ahk2* mutant germinated somewhat earlier than wild type, a trait which was enhanced in *ahk3* and *ahk2 ahk3* mutants. 15 h after sowing 50% of *ahk3* and *ahk2 ahk3* mutant seedlings had germinated, indicating a negative regulatory role of AHK3 in timing of seed germination. As a positive control seeds of *35S:CKX* transgenic cytokinin-deficient plants genes were analyzed. Seeds overexpressing *AtCKX2* or *AtCKX4* showed early germination similar to the receptor mutants (Fig. 6A and data not shown). Then it was tested whether the earlier seed germination could be retarded on medium containing cytokinin. Increasing concentrations of cytokinin caused later seed germination in the mutant seeds (data not shown), indicating i) that the trait is indeed caused by a reduced cytokinin status and ii) that in each of the double mutants the remaining receptor was able to transmit the cytokinin signal to regulate germination. Consistent with a combined activity of all three receptors, triple mutants germinated even earlier(Fig. 6A). A similar duration of seed rest after harvest was required for wild type and all single and double mutants, and all three showed a similar sensitivity to ABA (data not shown), indicating that regulation of an ABA-independent pathway is affected by reduced cytokinin signaling. Wild type *Arabidopsis* seeds of most ecotypes require light for efficient germination and action of active phytochrome is considered the primary event in seed germination. 5 days after sowing and continuous incubation in the dark only ca. 25% of wild type seeds had germinated (Fig. 6B). In contrast, 42% of the *cre1* seeds, more than 60% of the *ahk2 ahk3* and *ahk3 cre1* double mutant seeds, and ca. 80% of the *ahk2 cre1* double mutant and triple mutant seeds had germinated at this time point (Fig. 6B). This indicates a combined activity of all receptors to suppress germination of *Arabidopsis* seeds in the dark, the largest contributions coming from AHK2 and CRE1/AHK4. Germination is inhibited by far-red light and the active red light-induced Pfr form of phytochrome is required to trigger the germination pathway. It was therefore compared the germination under red and under far-red light. Fig. 6C shows that wild type and all mutant plants showed differences in timing of germination in red light where noted in white light (Fig. 6A) but comparable rates of germination after a few hours of red light treatment. Far-red light treatment inhibited germination in wild type, *ahk2* and *cre1* single mutants as well as *ahk2 cre1* double mutants (Fig. 6D). By contrast, more than 60% of all progeny that were homozygote for a mutated *ahk3* allele germinated. Also, all other mutant combinations involving a mutated *ahk3* allele showed resistance to far red light (Fig. 6D).

### 1.7: Root Phenotype of Cytokinin Receptor Mutants

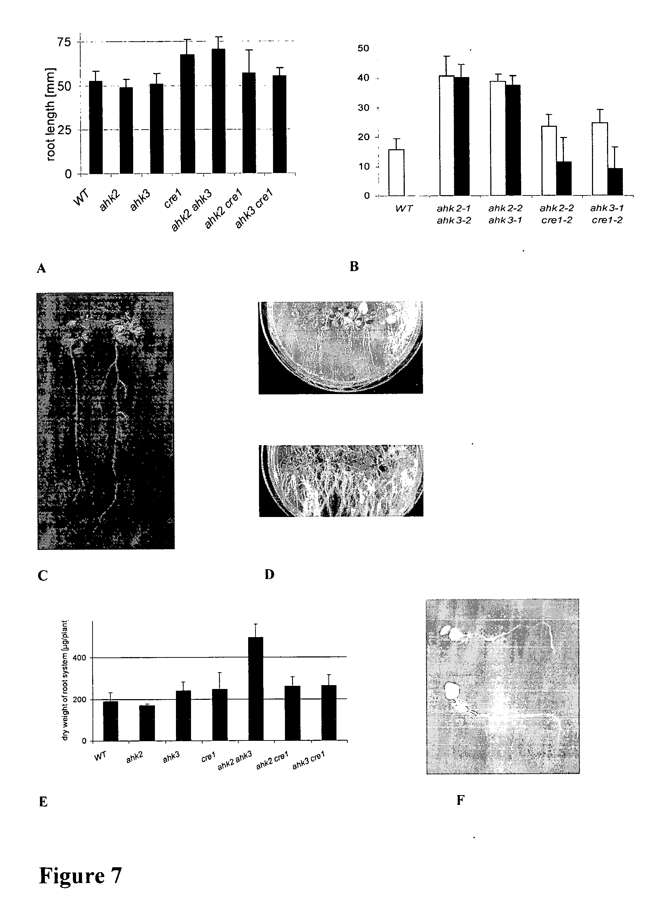

Ten days after germination the primary roots of *cre1* and the *ahk2 ahk3* double mutant were longer than in wild type (Fig. 7A). A similar increase in root elongation was found when the growth was measured in the interval between 4 DAG and 8 DAG (data not shown). Three weeks after germination all three double mutants had formed a larger number of lateral roots, while no significant difference was found for single mutant plants (Fig. 7B and data not shown). The greatest increase was found for *ahk2 ahk3* mutants which had more than twice the number of side roots compared to wild type. Furthermore, all double mutant plants, and in particular *ahk2 ahk3* plants, had formed secondary lateral branches, which were completely absent in wild type (Figs. 7B,C). As a consequence, *ahk2 ahk3* mutant plants formed a dense and highly branched root system (Fig. 7D). Three weeks after germination the dry weight of the root system of *ahk2 ahk3* mutants was ca. 250% of the wild type, while the enhancement in the other double mutant combinations was in the range of 20% (Fig. 7E). Recently it was hypothesized that cytokinin derived from the root cap is involved in regulating root gravitropism, presumably by inhibiting growth on one side of the root. If cytokinin had a central role in mediating root gravitropism one would expect an altered gravitropic response in cytokinin receptor mutants. Analysis of the root gravitropic response in all single mutants and mutant combinations revealed no difference between wild type and the receptor mutants. A normal positive gravitropic response was also seen in the triple mutant (Fig. 7F) indicating that cytokinin responsiveness is not required for gravitropism.

### 1.8: Conclusions

A detailed loss-of-function mutant analysis to study the roles of three cytokinin receptors in development and their participation in a variety of cytokinin-dependent processes was carried out. The general outcome is that the signal perception system is redundant, with all three receptors participating in most of the analyzed reactions. However, the three receptors and their combinations contribute to a different extent in different processes. One example is root branching, which was enhanced in all double mutants but particularly strong in the *ahk2 ahk3* combination (Fig. 7). This and other examples will be discussed below. The results will be, whenever possible, compared to the consequences of cytokinin-deficiency in Arabidopsis and tobacco. Cytokinin-deficiency was obtained by overexpression of *CKX* genes, which code for cytokinin-degrading cytokinin oxidases/dehydrogenases. Comparisons will also be made with previous reports on cytokinin receptor mutants, which analyzed some aspects of single mutants and specific combinations of multiple mutants. All three receptors participate in regulating shoot development. Mutations in single receptors did not cause strong changes of shoot growth indicating a high degree of redundancy of receptor functions in shoot growth regulation. The only phenotype we noted was a slight reduction of rosette size in *ahk3* mutant plants. Redundancy was not complete as combined loss of AHK2 and AHK3 restricted shoot growth, specifically chlorophyll and leaf cell formation. This shows that CRE1/AHK4 alone does not support all cytokinin functions in the shoot, while these functions where maintained by either AHK2 or AHK3 alone. In contrast, the rate of leaf initiation and the timing of flower induction in *ahk2 ahk3* mutants were similar to wild type, indicating that CRE1/AHK4 is sufficient to transmit the cytokinin signal for these processes. Consistently, introgression of the *cre* mutation in the *ahk2 ahk3* mutant slowed leaf formation and caused retarded flowering. Interestingly, changed leaf size did not alter overall leaf shape and heteroblasty, indicating that these traits are regulated independent of cytokinin. These alterations are generally in accordance with the shoot phenotype of cytokinin-deficient Arabidopsis plants. However, an important difference is that a strong reduction of the cytokinin content led to complete arrest of growth of the apical shoot meristem while triple receptor mutants are still able to establish and maintain a functional shoot meristem. In order to explain this discrepancy it is proposed that two separate cytokinin response systems exist. A second system that operates independently from the receptors should support basic cell cycle functions ensuring the formation of a sufficient number of cells for a basic plant body. This system may operate in an autocrine, i.e. cell-autonomous, fashion. Each cell's own cytokinin may act on the cell cycle machinery in the producing cell and thus make it independent from an extracellular cytokinin signal. Lack of cytokinin signaling via membrane-located receptors would not affect the basic maintenance function. In contrast, enhanced degradation of endogenous cytokinin by CKX, which depletes intra- and/or extracellular pools of cytokinin, depending on the subcellular localization of the enzyme, would lead to cessation of growth if the internal cytokinin pool is reduced. Consistent with this proposal, cessation of growth of shoots was only obtained when a CKX enzyme located in the vacuole was overexpressed, while overproduction of CKX enzymes located in the ER and/or extracellularly caused only weak shoot phenotypes and did not lead to cessation of growth (Werner et al., 2001, 2003). Thus the predicted receptor-independent maintenance function of cytokinin appears to depend on an intracellular hormone pool. A molecular mechanism to realize cytokinin functions in the cell cycle, independent of membrane-located receptors, could be a direct regulation of enzymatic activity. In accordance with this proposal is the fact that cytokinin compounds and structural derivatives efficiently modulate the activity of cell cycle-regulating kinases. In this model the main function of cytokinin perception and signaling via the two-component system would be to mediate information arriving from elsewhere in the plant or from the plant's environment and would serve to modulate growth in response to these cues.

AHK2 and AHK3 mediate Cytokinin-dependent senescence retardation. Cytokinin delays senescence in detached leaves. Loss of chlorophyll is one of the most obvious signs of leaf senescence and it was used here to determine that AHK3 has the most important role in mediating cytokinin-dependent chlorophyll retention in dark-treated leaves (Fig. 7). AHK3 alone was sufficient to mediate the full response. AHK2 or CRE1/AHK4 alone only weakly transmitted the cytokinin signal or did not transmit it at all. However, both receptors together provided full cytokinin responsiveness, at least at higher cytokinin concentrations (Fig. 7). The alternative possibility that CRE1/AHK4 is not present in leaf cells is less likely as analysis of a promoter-GUS fusion gene has shown that CRE1/AHK4 is expressed in leaf cells. What does this tell us about the role of cytokinin in *in planta* senescence? Several observations should be considered. First, the chlorophyll content of several cytokinin receptor mutant combinations was reduced compared to wild type, similar to cytokinin-deficient plants. This clearly indicates that the full cytokinin level or the full signaling competence is required to achieve wild type levels of chlorophyll. However, a basic level of chlorophyll is formed independent of cytokinin indicating that cytokinin is per se not necessary for chlorophyll formation but may function to modulate the chlorophyll content above the lower threshold level in response to environmental cues. The kinetics of loss of chlorophyll during senescence in detached leaves was similar in wild type and *ahk2 ahk3* mutants, indicating that the endogenous cytokinin in wild type does not retard the process. It could be that a low cytokinin content or reduced cytokinin signaling are not triggering factors for the onset of the senescence process but that a low cytokinin content is a licensing factor for senescence to occur. Consistent with this idea we have not noted earlier leaf senescence in cytokinin receptor mutants or cytokinin-deficient plants.

Cytokinin controls seed size. Despite its agronomic importance not much is known about the factors regulating seed size and a possible role for cytokinin has only recently been reported. Control of seed size involves control of growth in the embryo, the surrounding triploid endosperm and the seed coat. In Arabidopsis, the seed coat and endosperm growth precede embryo growth and the seed almost reaches its final size before the enlargement of the embryo, which happens during the later phase of embryogenesis. Genetic studies of others have shown that maternal and non-maternal factors are involved in seed size regulation and that crosstalk occurs between maternal and zygotic tissue to coordinate seed size. Genetic analysis of cytokinin receptor mutants has indicated that the increase of triple mutant seed size does not depend on the genotype of the embryo or of the mother plant, but rather is governed by the genotype of the endosperm. Analysis of increase of seed size in cytokinin-deficient plants has confirmed that this trait is not linked to the embryonic genotype. The endosperm acts as a sink for nutrient unloading from the phloem and is essential for reserve storage in the embryo cotyledons. The relevance of the endosperm is illustrated by several mutants, including *titan3, demeter, spätzle* and *haiku* published by others, which show premature arrest of endosperm development and, as a consequence, reduced seed size. Recently it was shown by others that mutations in the transcript factor gene *APETALA2 (AP2)* act maternally and/or in the endosperm to increase seed size. In addition, dosage effects of maternal and paternal tissue on endosperm development and seed size have suggested epigenetic mechanisms of seed size control. It is an important question whether the increased seed size is causally linked to the reduced number of seeds. It is conceivable that reduced availability of sink organs for fixed carbon may lead to an enhanced deposition in the available sink tissues, i.e. embryos, and it is known that the seed mass is generally negatively correlated with the total seed yield in Arabidopsis. It was shown by hand pollination of only few flowers of male sterile mutant plants or removal of all flowers on wild type plants with the exception of three flowers that were left for self-pollination that in Arabidopsis a reduction of seed number causes a mass increase in the individual seed of 22-33%. This is far lower than the 250% increase in size of the triple mutant seeds. A study of different Arabidopsis ecotypes also showed that reduced fertility has only a limited influence on seed size. The present inventors now conclude that the increase of seed size in the triple cytokinin receptor mutant is a direct consequence of loss of receptor functions. The mechanism through which the reduced cytokinin status of the endosperm enhances seed size is unclear but the formation of a greater number of endosperm cells is a plausible hypothesis. Consistent with this idea is the formation of smaller seeds by mutants with fewer endosperm cells.

Cytokinin controls seed germination. Arabidopsis seeds develop dormancy during the late stages of maturation, a process that depends on ABA. Breakage of dormancy and seed germination is primarily controlled by a reversible red light dependent equilibrium of the photoreceptors PHYA and PHYB. PHYB is required to suppress seed germination in the dark and PHYA suppresses seed germination in far-red light. One additional important factor to alleviate ABA-induced dormancy and germinate is GA, which is formed as a consequence of light action. Miller (1956) found that cytokinin in the dark could replace red light to induce seed germination in lettuce, but it was believed that cytokinin at physiological levels hardly affects seed germination and probably plays no role. The reduced dormancy of cytokinin receptor mutant seeds and cytokinin-deficient seeds, which becomes apparent as a more rapid germination, increased dark germination and reduced far-red light sensitivity, proves the relevance of the hormone in regulating this process. Both dark germination and far-red light sensitivity are altered in receptor mutants, indicating that cytokinin may control the PHYA and the PHYB pathways. Increased resistance to PHYA-dependent inhibition of germination by far-red light and increased dark germination are likely due to different mechanisms. Far-red light resistance indicates that cytokinin is required for production of an active PHYA, possibly involving signaling via ARRs as was reported for PHYB dependent hypocotyl elogation (see below). The red light absorbing form of PHYA would need to be stabilized by cytokinin (perhaps by blocking conversion to the active Pfr form). An alternative possibility to explain the dark germination of receptor mutants is an enhanced GA pathway due to lack of cytokinin. Cytokinin reduces the activity of GA in several bioassays and regulates expression of genes involved in GA synthesis and signaling. Consistent with enhanced GA activity is the fact that seed treatment with GA partially overcomes the inhibition of dark germination and the more rapid germination of the receptor mutant seeds. Our experiments do not distinguish between a PHYB and a GA-dependent effect. It is noteworthy that different receptors contribute differently to mediate the cytokinin effect. Mutation of CRE1/AHK4 caused the greatest enhancement of germination in the dark while mutation of AHK3 caused a partial resistance to far-red light. Together the data show that cytokinin is, under normal physiological conditions, a negative regulator of seed germination and that different independent pathways are controlled through different receptors.

Cytokinin regulates root architecture. The enhanced root system of *ahk2 ahk3* mutants is achieved in two ways. The primary root is growing faster than in wild type plants, which is comparable to the consequences of a reduced endogenous cytokinin content. Secondly, the *ahk2 ahk3* mutants form more lateral roots than wild type (Fig. 7). The formation of primary lateral roots was enhanced as well as the formation of secondary and tertiary lateral roots, which did not occur in wild type. This led to a significantly stronger root enhancement than did the reduction of the cytokinin content. The prominent role of AHK2 and AHK3 in regulating root growth is a bit surprising as the analysis of cytokinin resistance of mutant roots and elongation of the primary root had shown a major role for CRE1/AHK4 in roots. This indicates that the resistance to exogenous cytokinin, the regulation of primary root elongation and root branching are separate functions and that separate cytokinin receptors are involved in regulating these functions. Lateral roots in Arabidopsis originate from pericycle cells. Various hormones, mainly auxin, but also ethylene, brassinosteroids, abscisic acid, as well as different nutrients such as nitrate, phosphate, sulfate and iron regulate lateral root formation. A critical event in lateral root formation is re-entry of differentiated pericycle cells present in the primary root or previously formed lateral roots into the cell cycle and re-initiation of the root-tissue programme. From the present result it appears that cytokinin at physiological levels suppresses induction of cell division in the root pericycle and that this function is redundantly regulated by AHK2 and AHK3. Paradoxically, cytokinin would normally prevent re-entry of cells into the cell cycle although the hormone is usually considered to be a positive regulator of the cell cycle. Consistent with a very early activity of cytokinins in precursor cells of lateral roots is the observation that initiation of lateral roots is associated with a localized repression of a cytokinin-responsive reporter gene, indicating spatial and temporal regulation of the cytokinin status during lateral root formation. However, it should be noted that the initial cell divisions are required for lateral root formation but it is not the trigger for lateral root development. Therefore, not only the cell cycle but also the further developmental program needs to be controlled by cytokinin. It is hypothesized by the present inventors that physiological levels of cytokinins are superoptimal for maximal root elongation and initiation of the lateral root formation program. Optimal conditions may be achieved by decreasing the endogenous cytokinin content or by decreasing cytokinin signaling. This model provides a basis for crosstalk between other effector molecules and cytokinin. For example, locally enhanced nitrate concentration enhances root branching locally. Nitrate induces A-type response regulator genes. Enhanced levels of A-type response regulators would reduce cytokinin signaling and thus facilitate the formation of side roots. Together these data corroborate the relevance of the cytokinin status in regulating root architecture. They also argue against the recently proposed role of cytokinin in regulating the gravitropic response.

The analyses of the present invention have yielded novel information about the involvement of different receptors and their combinations in various cytokinin-regulated processes. Unexpectedly, they revealed various functions of cytokinin in seed biology.
Many of the responses are driven by multiple cytokinin receptors in an additive manner. In other cases, the contribution of a given receptor could only be identified in the absence of others. It is noteworthy that mutation of *AHK2* did not cause a significant change of cytokinin sensitivity in any of the tests. However, the *ahk2* mutation enhanced cytokinin resistance of *ahk3* and *ahk4* mutants. This indicates that AHK2 may function primarily in combination with AHK3 and CRE1/AHK4, while the latter two do not seem to cooperate. One possibility is the formation of AHK2-AHK3 and AHK2-CRE1/AHK4 receptor heterodimers, which can be tested experimentally. These relationships could be the result of parallel pathways or interaction between pathways, which cannot be distinguished by these experiments. For example, homo- and heterodimerization could allow the differential responses and coupling to different intracellular pathways in response to a single elicitor. Moreover, receptor homodimers and heterodimers could detect different cytokinins with different sensitivity. Indeed, analysis of the present inventors in a yeast two-hybrid system has indicated that specific interaction between different cytokinin receptors takes place. Lack of additive effects or disappearance of the effects of one mutation in the presence of a second mutation may be explained by partial or complete suppression of one mutation by another mutation which was also found in mutants that carry mutations in more than one photoreceptor. Our data provide substantial support for the concept of a function of cytokinins in positive control of shoot development and negative control of root growth. Further work has to show how the cytokinin receptors are linked downstream to different signaling pathways in order to achieve positive or negative regulatory control on the cell cycle and/or exit of cells from the meristems. It is noteworthy that several of the cytokinin-regulated traits are of agronomic importance. Water and nutrient availability limit yield in most agricultural ecosystems. Improved root systems are therefore of considerable interest in agriculture. Control of senescence is relevant for yield and shelf life. Last but not least, seed size profoundly influences total harvest.

Following the figures of this example are explained in more detail.

Figure 1 shows the identification and molecular characterization of *ahk2* and *ahk3* mutant alleles. (A) shows structures of the *AHK2* and *AHK3* genes and insertion sites of the T-DNAs in the *ahk2* and *ahk3* mutant alleles used. Thick lines represent exons. T-DNA insertions are indicated as triangles and are not drawn to scale. (B) shows the sequence at the genome-T-DNA junction sites in the *ahk2* and *ahk3* mutant alleles. The sequences of the genes are shown on a gray background, the sequence of the T-DNA is printed in bold, sequences of unknown origin found in two of the insertion lines are underlined. Numbers above the sequence indicate the base pair position relative to the predicted translational start codon of the respective gene. (C) shows a RT-PCR analysis of *AHK2* and *AHK3* transcripts. Primers specific for the *AHK2* transcript and the *AHK3* transcript were used to analyze the *ahk2-5* and *ahk3-6* mutants, respectively. The *ahk2-5, ahk3-6* double mutant was analyzed with primers for both genes. In all cases the upper band corresponds to the *actin2* transcript, which was used as a positive control. M, 1 kb ladder, the size in bp is indicated on the right.

Figure 2 shows that the cytokinin sensitivity of cytokinin receptor mutants is decreased in shoots and roots. (A) shows reduced callus formation and regeneration capacity of *ahk2* and *ahk3* mutant hypocotyls on media containing different auxin (NAA) and cytokinin (2iP) concentrations. The hypocotyl segments of seven-day-old plants were cut off and cultured for 28 days on different hormone media as shown. Representative samples for each tested concentration were placed together prior to photography. (B) shows enhanced growth, leaf greening and flowering capacity of *ahk2 ahk3* double mutants compared to wild type (WT) and single mutants on medium supplemented with cytokinin (40 ng/ml BA). Photo was taken 28 DAG. (C) shows elongation of the primary root of wild type (WT) and mutant plants between 4 DAG and 7 DAG on media containing different concentrations of BA. Root elongation on cytokinin-free medium was set at 100%. Root elongation on this medium was for WT 1.06 ±0.17 cm; *ahk2,* 1.05 ±0.18 cm; *ahk3,* 1.06 ±0.16 cm; *ahk2 ahk3,* 1.42 ±0.38 cm; *ahk2 cre1,* 0.86 ±0.27 cm; *ahk3 cre1,* 1.21 ±0.25 cm and *ahk2 ahk3 cre1,* 1.24 ±0.32 cm.
(D) Elongation of the primary roots of *ahk2 ahk3* mutants (two seedlings on the right) compared to wild type on MS medium containing 100 ng/l BA 27 DAG.

Figure 3 shows shoot development of *ahk* mutant plants. (A) shows rosette sizes of WT and cytokinin receptor mutants 25 DAG. (B) shows rosettes of plants grown on soil 25 DAG. Bar size is 1 cm for the close-up of the *ahk2 ahk3 crel* triple mutant shown in the lower right. (C) shows plants at the flowering stage 42 DAG. From left to right, WT, *ahk2, ahk3, ahk2 akh3.* (D) shows a triple *ahk2 ahk3 cre1* mutant plant (right) 70 DAG compared to WT. (E) shows downward bending of cotyledons. (F) shows leaves of WT (top row), *ahk2 cre1* (second row), *ahk3 cre1* (third row), *ahk2 ahk3* (bottom row, left), and of the triple mutant (bottom row, right) 25 DAG. The leaves were sorted according to age with the two cotyledons and the oldest leaves on the left. (G) shows number of epidermal cells per mm² on the adaxial (white column) and abaxial side (black column) of the seventh leaf 21 DAG. Cell size was measured at three different positions on the fifth leaf in the middle of the leaf blade. (H) shows number of epidermal cells on the seventh leaf. (I) shows an *ahk2 ahk3 wol* mutant plant compared to *wo1* and *ahk2 ahk3 cre*

Figure 4 shows that AHK2 and AHK3 are required to mediate cytokinin-dependent chlorophyll retention in the dark. (A) shows chlorophyll content of in vitro grown plants 24 DAG. Wild type was set at 1. For each of five independent samples per clone five leaves (the seventh leaf) from five different plants were pooled. (B) shows dark-induced senescence in a leaf floating assay and its inhibition by cytokinin. The leaf chlorophyll content before the start of dark incubation was set at 1 for each genotype tested. Columns: white, water plus DMSO; gray, 0.1 µM BA; black, 1µM BA. Chlorophyll A content (µg/g leaf fresh weight) at the beginning of the assay: *ahk2 ahk3* (1), 0.63 ±0.03 SD; *ahk2 ahk3* (2), 0.65 ±0.04 SD; *ahk2 cre1,* 1.09 ±0.05 SD; *ahk3 cre1,* 0.67 ±0.02 SD; *ahk2 ahk3 cre1,* 0.30 ±0.02 SD. (C) shows leaves of different genotypes at the end of the chlorophyll retention assay described in B. (D) shows time-course of dark-induced leaf senescence in WT leaves. The empty squares show the chlorophyll content after incubation in water and the solid squares after incubation in water supplemented with 1 µM BA. (E) shows time-course of dark-induced leaf senescence in leaves of the *ahk2 ahk3* mutant. Conditions are as described in D (triangles were used instead of squares).

Figure 5 shows that seeds and embryos of the *ahk2 ahk3 cre1* Triple Mutant are Increased in Size. (A) shows *ahk2 ahk3 cre1* triple mutant seeds (upper two seeds) compared to WT. (B) shows embryos of the *ahk2 ahk3 cre1* triple mutant (top) and WT (bottom). (C) shows width (white bars) and length (black bars) of seeds from WT and the triple mutant. (D) shows calculated volume of wild type and triple' mutant seeds.

Figure 6 shows that seeds of cytokinin receptor mutants show early germination, a reduced requirement for light to germinate and resistance to far-red light. (A) shows the percentage of germinated seeds 24h after plating and incubation under long day conditions on cytokinin-free medium (white columns). B) shows the percentage of seeds germinated after five days incubation in the dark on MS medium.
(C) Germination rates under continuous red light after 24 h and after 48 h. (D) shows the percentage of germinated seeds after three days incubation under constant far red light.

Figure 7 shows that *ahk2 ahk3* double mutants form an enhanced root system. (A) shows the elongation of primary roots 11 DAG. (B) shows the number of lateral roots of first order (gray columns) and second order (black columns).(C) shows the root system of *in vitro* grown WT (left) and *ahk2 ahk3* mutant plants 14 DAG. (D) shows the enhanced root system of ahk2 ahk3 mutants plants grown in vitro for 28 days on vertical plates (bottom) compared to WT (top). (E) shows the dry weight of the root system 28 DAG. The root system was harvested 28 DAG, the roots of eight plants were pooled and the dry weight of four independent pools per genotype was determined. (F) shows the geotropic growth in WT (top) and the *ahk2 ahk3 cre1* triple mutant. Seeds were germinated on vertical plates and turned 90° 7 DAG. The pictures were taken 4 days later.

### Example 2: transgenic plants of the invention with inhibited or reduced expression of AHK2/AHK3 receptors by means of RNA inhibition.

### 2.1: Example to increase seed mass of plants

To generate plants with larger seeds in Arabidopsis the three cytokinin receptor genes AHK2, AHK3 and AHK4 are downregulated by means of antisense or RNAi constructs, which are directed against the CHASE-domain of the cytokinin receptor genes and are preferentially expressed in the endosperm and embryo. Reduced expression is achieved either by simultaneous cotransformation of three CHASE-domain RNAi/antisense constructs for *AHK2, AHK3* and *AHK4* or by transformation of one RNAi/antisense construct consisting of characteristic sequences all three CHASE-domains which are combined by ligation to a single construct. In the following the different constructs and their effects in transgenic plants are described.

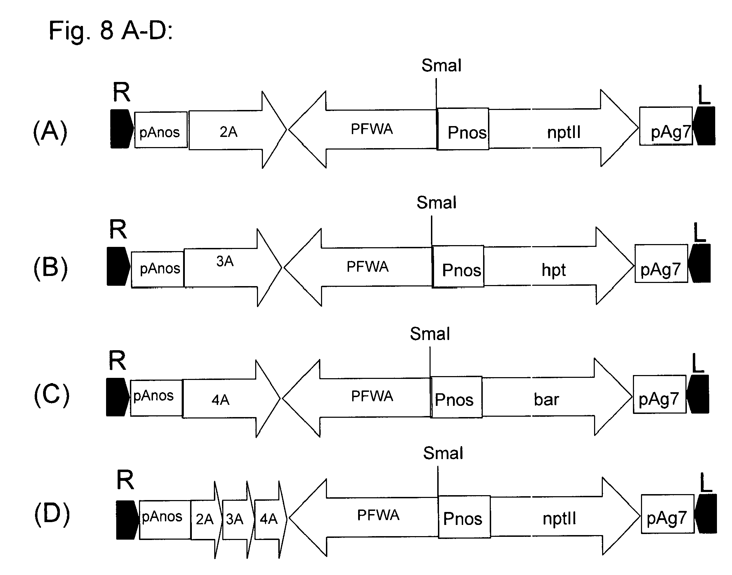

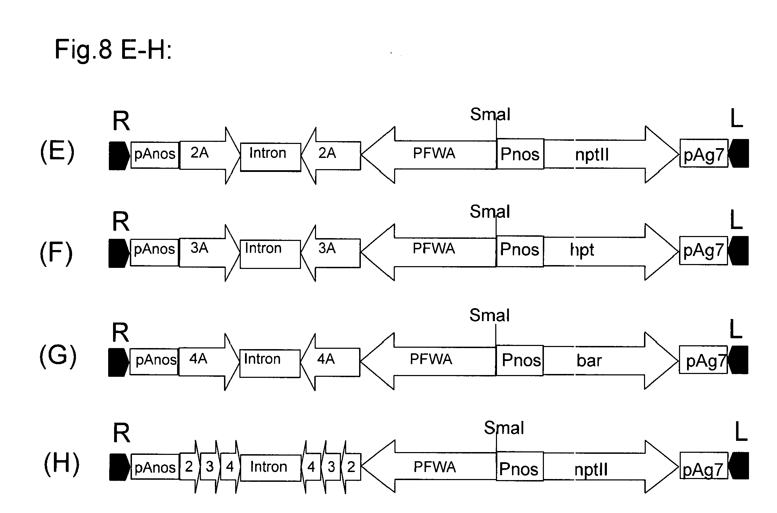

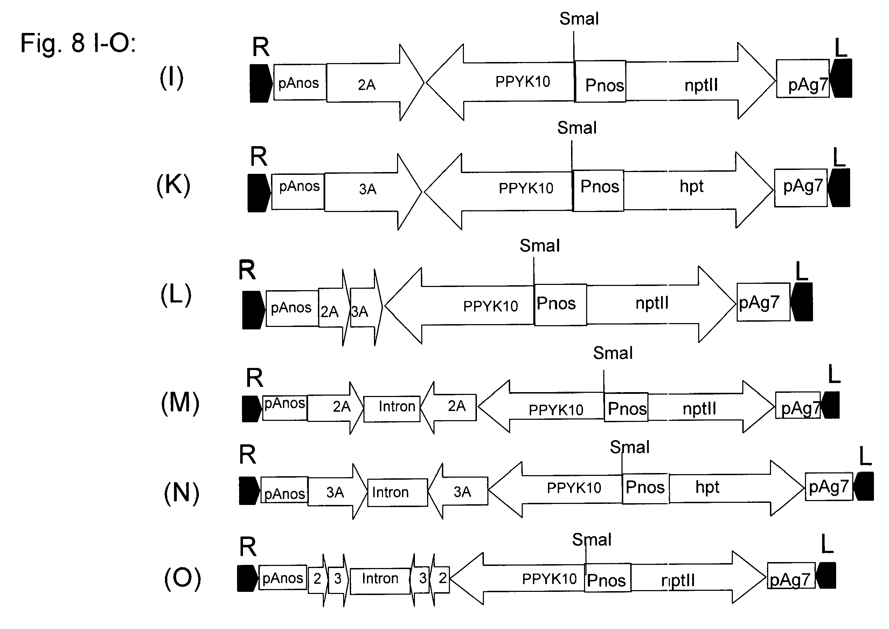

As a general note for the modification of pGPTV binary vectors the following is mentioned. The uidA gene of pGPTV is deleted at its SstI and SmalI sites. Overhang of SstI restriction site is digested with ExonucleaseI for blunt end cloning. Details of the T-DNA-constructs within the binary vector pGPTV-series are shown scematically in Figure 8. Therein are shown:. (A) pPF-A2as, (B) pPF-A3as, (C) pPF-A4as, (D) pPF-A234as, (E) pPF-A2ri, (F) pPF-A3ri, (G) pPF-A4ri, (H) pPF-A234rix, (I) pPP-A2as, (K) pPP-A3as, (L) pPP-A23as, (M) pPF-A2ri, (N) pPF-A3ri, (O) pPP-A23rix. L, left border of T-DNA; R, right border of T-DNA; 2A, *AHK2* CHASE-domain (~600bp); 3A, *AHK3* CHASE-domain (~600bp); 4A, *AHK4* CHASE-domain (~600bp); 2, *AHK2* CHASE-domain segment (~200-250bp); *3, AHK3* CHASE-domain segment (~200-250bp); 2, *AHK4* CHASE-domain segment (~200-250bp); intron, intron of RNAi construct of pHellsgate2 binary vector; *PFWA, FWA* promoter; *PPYK10, PYK10* promoter; Pnos, promoter of nopalin synthase gene; pAnos, polyadenylation signal of nopalin synthase gene; nptII, resistance to kanamycin; hpt, resistance to hygromycin; pAg7, polyadenylation signal; SmaI, SmaI restriction site; arrows indicate direction of transcription; sequences are not drawn to scale.

### 1.A. Construction and cotransformation of AHK2, AHK3 and AHK4 antisense constructs

### Step1:

Cloning of the endosperm-specific promotor in pUC19. As an example cloning with the FWA promotor (Acc. No.: AT4G25530; Luo et al., 2000) is described. Alternatively the FIS2 promoter may be used for the experiments (Kinoshita et al., 2004).
pFWA-forward: HindIII-SmaI-TTAATATTATGGGAAGGAGT
pFWA-reverse: PstI -XhoI-TTTCCCTCAATGCAATAACCT

This amplifies the FWA promotor (of 2104 bp length) in a PCR reaction. Recognition sites are added to the primers as indicated. HindIII and PstI and used for directional cloning into the multiple cloning site of pUC19 yielding pUC19-PF.

### Step2:

Directional PCR-based amplification and cloning of the single *AHK2, AHK3* and *AHK4* constructs (each 600-650 bp long) in three independent pUC19-PF vectors (described in step 1) using BamHI and XhoI. This results in plasmids pUC19-PF-A2as, pUC19-PF-A3as and pUC19-PF-A4as.
pUC19-*AHK2*-forward: BamHI- TCTGCCATTGATCAGAGAACA
pUC19-*AHK2*-reverse: XhoI- TCCCCAATTTCTGAGCCATA
pUC19-*AHK3*-forward: BamHI- GCTATGAGGGTGCTCCATTC
pUC19-*AHK3*-reverse: XhoI - CGCATCTCATGCTTTCTCAA
pUC19-*AHK4*-forward: BamHI- TGGAGTGGCTTATGCTGAAA
pUC19-*AHK4*-reverse: XhoI - AACGGTATTGGTGCCTTTTG

### Step3:

Subcloning of the FWA promoter-antisense fusion construct in a binary vector of the pGPTV series (Becker et al., 1992) using the SmaI restriction site for insertion. For the *AHK2, AHK3* and *AHK4* antisense constructs the vectors pGPTV-KAN, pGPTV-HPT and pGPTV-BAR are used respectively. This yields plasmids pPF-A2as, pPF-A3as and pPF-A4as.

### Step4:

pPF-A2as, pPF-A3as and pPF-A4as are transformed in three independent cultures of the *Agrobacterium* strain GV3101 (Koncz and Schell 1986). The next step is cotransformation of the three T-DNAs via the flower dip vacuum infiltration method in *Arabidopsis thaliana* and selection of multiple transformants on medium containing kanmycin, hygromycin and phosphotricine.

### 1.B. Construction and transformation of an antisense construct consisting of three consecutive segments of the CHASE-domains of AHK2, AHK3 and AHK4

PCR products of the three CHASE-domain segments (~ 600-650 bp each) are fused to a combined construct consisting finally of all three segments. The fusion is accomplished in three consecutive directional cloning steps in pUC19 harbouring the FWA promoter (see example 1.A.). This construct consisting of the FWA promoter and the three antisense oriented segments of the CHASE-domain of *AHK2, AHK3* and *AHK4* is transformed into Arabidopsis.

### Step1:

A PCR with chosen segments of the CHASE-domains using the pUC19-3-primers listed below harbouring additional specific restriction sites is carried out. This amplifies PCR-products of 600-650 bp for *AHK2, AHK3* or *AHK4* spanning almost the whole CHASE-domains.

Used primers are:
pUC19-3-*AHK2*-forward: XhoI- TCTGCCATTGATCAGAGAACA
pUC19-3-*AHK2*-reverse: XhoI -AvrII- TCCCCAATTTCTGAGCCATA

The resulted PCR-product is cloned in antisense orientation in the XhoI site of pUC19-PF (described in step 1 of example 1.A.) yielding pUC19-PF-A2as.

### Step2:

pUC19-3-*AHK3*- forward: AvrII - GCTATGAGGGTGCTCCATTC
pUC19-3-*AHK3*-reverse: AvrII - AscI- CGCATCTCATGCTTTCTCAA

The resulting PCR product is cloned in antisense orientation in the AvrII site of pUC19-PF-A2as yielding pUC19-PF-A23as.

### Step3:

pUC19-3-AHK4- forward: AscI - TGGAGTGGCTTATGCTGAAA
pUC19-3-*AHK4*-reverse: AscI - AACGGTATTGGTGCCTTTTG

The resulting PCR product is cloned in antisense orientation in the ASCI site of pUC19-PF-A23as yielding pUC19-PF-A234as.

### Step4:

The insert of pUC19-PF-A234as is subcloned into the SmaI site of the binary plant vector pGPTV-KAN (Becker et al., 1992) yielding plasmid pPF-A234as.

### Step5:

pPF-A234as is transformed in *Agrobacterium* strain GV3101 followed by transformation of Arabidopsis by the flower dip vacuum infiltration method.

### 2.A. Construction and cotransformation of AHK2, AHK3 and AHK4 RNAi constructs

pHellsgate2 (Wesley et al., 2001) is used to create the RNAi constructs. Then the RNAi constructs are subcloned in pUC19 vector containing the FWA promoter (pUC19-PF, see above). The resulting fusion genes are subcloned into binary vectors (pGPTV series; Becker et al., 1992) and finally transferred to Arabidopsis.

### Step1:

Construction of pUC19-PF. See example 1.A. above.

### Step2:

Cloning of the single *AHK2, AHK3* and *AHK4* CHASE-domains in pHellsgate2 results in three independent constructs harbouring RNAi constructs.

First a PCR is carried out with the chosen segments of the CHASE-domains using primers listed below with attB-linker-sequences. This amplifies PCR products of 662 bp for *AHK2,* 654 bp for *AHK3* and 657 bp for *AHK4* spanning the major part of the CHASE-domains.

General design of primers with attB1- and attB2-Gateway linkers.
(attB1): 5'-GGGG-ACA-AGT-TTG-TAC-AAA-AAA-GCA-GGC-TGG-(gene-specific sequence)-3'
(attB2): 5'-GGGG-AC-CAC-TTT-GTA-CAA-GAA-AGC-TGG-GTG-(gene-specific sequence)-3'

Specific linkers for the PCR:
pHellsgate2-*AHK2*-forward: attB1- TCTGCCATTGATCAGAGAACA
pHellsgate2-*AHK2*-reverse: attB2- TCCCCAATTTCTGAGCCATA
pHellsgate2-*AHK3*-forward: attB1- GCTATGAGGGTGCTCCATTC
pHellsgate2-*AHK3*-reverse: attB2- CGCATCTCATGCTTTCTCAA
pHellsgate2-*AHK4*-forward: attB1- TGGAGTGGCTTATGCTGAAA
pHellsgate2-*AHK4*-reverse: attB2- AACGGTATTGGTGCCTTTTG

The recombination reaction (PB-reaction) with pHellsgate2 and attB-*AHK*-PCR products allows cloning into pHellsgate2 yielding pHG-A2ri, pHG-A3ri and pHG-A4ri, respectively.

### Step3:

The individual *AHK2, AHK3* and *AHK4* RNAi constructs are amplified by PCR from pHG-A2ri, pHG-A3ri and pHG-A4ri and subcloned in the BamHI site of pUC19-PF, yielding plasmids pUC19-PF-A2ri, pUC19-PF-A3ri and pUC19-PF-A4ri.
pUC19-*AHK2*-forward: BamHI-attB1- TCTGCCATTGATCAGAGAACA
pUC19-*AHK2*-reverse: BamHI-attB2- TCCCCAATTTCTGAGCCATA
pUC19-*AHK3*-forward: BamHI-attB1- GCTATGAGGGTGCTCCATTC
pUC19-*AHK3*-reverse: BamHI-attB2- CGCATCTCATGCTTTCTCAA
pUC19-*AHK4*-forward: BamHI-attB1- TGGAGTGGCTTATGCTGAAA
pUC19-*AHK4*-reverse: BamHI-attB2- AACGGTATTGGTGCCTTTTG

### Step4:

The inserts of pUC19-PF-A2ri, pUC19-PF-A3ri and pUC19-PF-A4ri were subcloned in the SmaI site of the binary vectors pGPTV-KAN, pGPTV-HPT and pGPTV-BAR. This yields the binary plant vectors pPF-A2ri, pPF-A3ri and pPF-A4ri.

### Step5:

Agrobacterium harbouring pPF-A2ri, pPF-A3ri and pPF-A4ri are used to cotransform Arabidopsis using the flower dip vacuum infiltration method. Transgenic plants are selected on media containing kanamycin, hygromycin and phosphinotricine to select for individuals harbouring all three T-DNAs.

### 2.B. Construction of RNAi-genes with three consecutive segments of the CHASE-domains of AHK2, AHK3 and AHK4 and generation of transgenic plants

PCR products of the three CHASE-domain segments (~200-250bp each) are fused to a combined construct consisting of all three segments. The fusion is accomplished in three consecutive directional cloning steps in pUC19-PF. The consecutive subcloning steps in pHellsgate2 and pUC19-PF produces a FWA-promoter-RNAi fusion gene. This fusion gene is transferred to a binary vector and subsequently transformed into Arabidopsis.

### Step1:

A PCR to amplify chosen segments of the CHASE-domains is carried out using pUC19-3-primers with additional specific restriction sites as indicated below. This yields PCR-products of 243 bp for *AHK2,* 231 bp for *AHK3* and 225 bp for *AHK4* spanning approximately a third of the respective CHASE-domains.

### Used primers are:

pUC19-3-*AHK2*-forward: XhoI - AGGGCATCAGGAAAAGGAGT
pUC19-3-*AHK2*-reverse: XhoI -AvrII- TGTCTGTTTGCTGGCAAGTT

The resulting PCR-product is cloned in antisense orientation in the XhoI site of pUC19-PF (see 1.A.) to yield pUC19-PF-A2rix.

### Step2:

pUC19-3-AHK3- forward: AvrII - GGTATCTCGGGGGAGTGTTT
pUC19-3-*AHK3*-reverse: AvrII - AscI-CGCATCTCATGCTTTCTCAA

The resulting PCR-product is cloned in antisense orientation in the AvrII site of pUC19-PF-A2rix yielding pUC19-PF-A23rix.

### Step3:

pUC19-3-*AHK4*- forward: AscI - AAGAGCGTATTGCAGCCACT
pUC19-3-*AHK4*-reverse: AscI - CCAAAATCGAGCTTGCTCTC

The resulting PCR-product is cloned in antisense orientation in the AscI site of pUC19-PF-A23rix to yield pUC19-PF-A234rix.

### Step4:

The insert downstream of the FWA promoter (i.e. the fused AHK2, AHK3, AHK4 CHASE segments) of pUC19-PF-A234rix is subcloned into pHellsgate2 by PCR-based cloning.

### Primers are:

pHellsgate2-3-forward: attB1- AscI- AGGGCATCAGGAAAAGGAGT
pHellsgate2-3-reverse: attB2- XhoI - CCAAAATCGAGCTTGCTCTC

Introduction of the construct in pHellsgate2 via recombination (PB-reaction) yields pHG-A234rix.

### Step5:

It follows a PCR-based cloning step with BamHI as linkers to reintroduce this RNAi construct into pUC19-PF (see 1.A.) downstream of the FWA promoter.
pUC19-31-forward: BamHI-attB1- AscI- AGGGCATCAGGAAAAGGAGT
pUC19-31-reverse: BamHI-attB2- XhoI - CCAAAATCGAGCTTGCTCTC

The resulted PCR-product is cloned in the BamHI site of pUC19-PF to yield pUC19-PF-A234rix-II.

### Step6:

The insert of pUC19-PF-A234rix-II is subcloned in the SmaI site of the binary vector pGPTV-KAN to yield pPF-A234rix.

### Step7:

Agrobacterium harbouring pPF-A234rix are used to transform Arabidopsis using the flower dip vacuum infiltration method. Transgenic plants were selected on media containing kanamycin.

### Evaluation of transgenic plants

For each of the above constructs at least ten different transgenic lines are obtained. Seed size and seed yield is surveyed in the T1 generation and homozygote T2 plants of the best two clones are analyzed in further detail. Shoot growth and fertility of these transgenic lines are not affected. Quantitative comparison of elongation of seed size and seed yield indicates a significant increase of seed size and seed yield in all cases. Irrespective of the construct used all transgenic lines show an increase in seed volume from 45% up to 250% and more. These results demonstrate that tissue-specific downregulation of the AHK2, AHK3 and AHK4 genes results in enhanced seed size and seed yield without detrimental effects on other plant parts.

### 2.2 Example to increase root growth of plants

To generate plants with enhanced root growth in Arabidopsis the three cytokinin receptor genes AHK2 and AHK3 are downregulated by means of antisense or RNAi constructs, which are directed against the CHASE-domain of these cytokinin receptor genes and are principally expressed in the root. Reduced expression is achieved either by simultaneous cotransformation of two CHASE-domain RNAi/antisense constructs for *AHK2* and *AHK3* or by transformation of one RNAi/antisense construct consisting of characteristic sequences both CHASE-domains which are combined by ligation to a single construct. In the following the different constructs and their effects in transgenic plants are described.

### 3.A. Construction and cotransformation of AHK2 and AHK3 antisense constructs

### Step1:

Cloning of the root-specific promotor in pUC19. As an example cloning with the PYK10 promotor (Acc. No.: AT3G09260; Nitz et al., 2001) is described.
pFWA-forward: HindIII-SmaI-TTAATATTATGGGAAGGAGT,
pFWA-reverse: BamHI GAACAG XhoI-TTTCCCTCAATGCAATAACCT

This amplifies the PYK10 promotor (of 2038 bp length) in a PCR reaction. Recognition sites are added to the primers as indicated. HindIII and BamHI and used for directional cloning into the multiple cloning site of pUC19 yielding pUC19-PP.

### Step2:

Directional PCR-based amplification and cloning of the single *AHK2* and *AHK3* constructs (each 600-650 bp long) in two independent pUC19-PP vectors (described in step 1) using BamHI and XhoI. This results in plasmids pUC19-PP-A2asand pUC19-PP-A3as.
pUC19-*AHK2*-forward: BamHI- TCTGCCATTGATCAGAGAACA
pUC19-*AHK2*-reverse: XhoI- TCCCCAATTTCTGAGCCATA
pUC19-*AHK3*-forward: BamHI- GCTATGAGGGTGCTCCATTC
pUC19-*AHK3*-reverse: XhoI - CGCATCTCATGCTTTCTCAA

### Step3:

Subcloning of the PYK10 promoter-antisense fusion construct in a binary vector of the pGPTV series (Becker et al., 1992) using the SmaI restriction site for insertion. For the *AHK2* and *AHK3* antisense constructs the vectors pGPTV-HPT and pGPTV-BAR are used respectively. This yields plasmids pPP-A2as and pPP-A3as.

### Step4:

pPP-A2as and pPP-A3as are transformed in two independent cultures of the *Agrobacterium* strain GV3101 (Koncz and Schell 1986). The next step is cotransformation of the two T-DNAs via the flower dip vacuum infiltration method in *Arabidopsis thaliana* and selection of multiple transformants on medium containing hygromycin and phosphotricine to select double transformants.

### 3.B. Construction and transformation of an antisense construct consisting of two consecutive segments of the CHASE-domains of AHK2 and AHK3

PCR products of the three CHASE-domain segments (~ 600-650 bp each) are fused to a combined construct consisting finally of all three segments. The fusion is accomplished in two consecutive directional cloning steps in pUC19 harbouring the PYK10 promoter (see example 3.A.). This construct consisting of the PYK10 promoter and the two antisense oriented segments of the CHASE-domain of *AHK2* and *AHK3* is transformed into Arabidopsis.

### Step1:

A PCR with chosen segments of the CHASE-domains using the pUC19-3-primers listed below harbouring additional specific restriction sites is carried out. This amplifies PCR-products of 600-650 bp for *AHK2* or *AHK3* spanning almost the whole CHASE-domains.

### Used primers are:

pUC19-3-*AHK2*-forward: XhoI- TCTGCCATTGATCAGAGAACA
pUC19-3-*AHK2*-reverse: XhoI -AvrII- TCCCCAATTTCTGAGCCATA

The resulted PCR-product is cloned in antisense orientation in the XhoI site of pUC19-PP (described in step 1 of example 3.A.) yielding pUC19-PP-A2as.

### Step2:

pUC19-3-AHK3- forward: AvrII - GCTATGAGGGTGCTCCATTC
pUC19-3-*AHK3*-reverse: AvrII - AscI- CGCATCTCATGCTTTCTCAA

The resulting PCR product is cloned in antisense orientation in the AvrII site of pUC19-PP-A2as yielding pUC19-PP-A23as.

### Step3:

The insert of pUC19-PP-A23as is subcloned into the SmaI site of the binary plant vector pGPTV-KAN (Becker et al., 1992) yielding plasmid pPP-A23as.

### Step4:

pPP-A23as is transformed in *Agrobacterium* strain GV3101 followed by transformation of Arabidopsis by the flower dip vacuum infiltration method. Transgenic individuals are selected on kanamycin containing medium.

### 4.A. Construction and cotransformation of AHK2 and AHK3 RNAi constructs

pHellsgate2 (Wesley et al., 2001) is used to create the RNAi constructs. Then the RNAi constructs are subcloned in the pUC19 vector containing the PYK10 promoter (pUC19-PP, see above). The resulting fusion genes are subcloned into binary vectors (pGPTV series; Becker et al., 1992) and finally transferred to Arabidopsis.

### Step1:

Construction of pUC19-PP. See example 3.A. above.

### Step2:

Cloning of the single *AHK2* and *AHK3* CHASE-domains in pHellsgate2 results in two independent constructs harbouring RNAi constructs.

First a PCR is carried out with the chosen segments of the CHASE-domains using primers listed below with attB-linker-sequences. This amplifies PCR products of 662 bp for *AHK2* and 654 bp for *AHK3* spanning the major part of the CHASE-domains.

General design of primers with attB1- and attB2-Gateway linkers.
(attB1): 5'-GGGG-ACA-AGT-TTG-TAC-AAA-AAA-GCA-GGC-TGG-(gene-specific sequence)-3'
(attB2): 5'-GGGG-AC-CAC-TTT-GTA-CAA-GAA-AGC-TGG-GTG-(gene-specific sequence)-3'

Specific linkers for the PCR:
pHellsgate2-AHK2-forward: attB1- TCTGCCATTGATCAGAGAACA
pHellsgate2-AHK2-reverse: attB2- TCCCCAATTTCTGAGCCATA
pHellsgate2-AHK3-forward: attB1- GCTATGAGGGTGCTCCATTC
pHellsgate2-AHK3-reverse: attB2- CGCATCTCATGCTTTCTCAA

The recombination reaction (PB-reaction) with pHellsgate2 and attB-AHK-PCR products allows cloning into pHellsgate2 yielding pHG-A2ri and pHG-A3ri.

### Step3:

The individual *AHK2* and *AHK3* RNAi constructs are amplified by PCR from pHG-A2ri and pHG-A3ri and subcloned in the BamHI site of pUC19-PP, yielding plasmids pUC19-PP-A2ri and pUC19-PP-A3ri.
pUC19-*AHK2*-forward: BamHI-attB1- TCTGCCATTGATCAGAGAACA
pUC19-*AHK2*-reverse: BamHI-attB2- TCCCCAATTTCTGAGCCATA
pUC19-*AHK3*-forward: BamHI-attB1- GCTATGAGGGTGCTCCATTC
pUC19-*AHK3*-reverse: BamHI-attB2- CGCATCTCATGCTTTCTCAA

### Step4:

The inserts of pUC19-PP-A2ri and pUC19-PF-A3ri are subcloned in the SmaI site of the binary vectors pGPTV-HPT and pGPTV-BAR. This yields the binary plant vectors pPP-A2ri and pPP-A3ri.

### Step5:

Agrobacterium harbouring pPF-A2ri and pPF-A3ri are used to cotransform Arabidopsis using the flower dip vacuum infiltration method. Transgenic plants are selected on media containing hygromycin and phosphinotricine to select for individuals harbouring all three T-DNAs.

### 4.B. Construction of RNAi-genes with two consecutive segments of the CHASE-domains of AHK2 and AHK3 and generation of transgenic plants

PCR products of the two CHASE-domain segments (~200-250bp each) of AHK2 and AHK3 are fused to a combined construct consisting of both segments. The fusion is accomplished in two consecutive directional cloning steps in pUC19-PP. The consecutive subcloning steps in pHellsgate2 and reintroduction in pUC19-PP produces a PYK.10-promoter-RNAi fusion gene. This fusion gene is transferred to a binary vector and subsequently transformed into Arabidopsis.

### Step1:

A PCR to amplify chosen segments of the CHASE-domains is carried out using pUC19-3-primers with additional specific restriction sites as indicated below. This yields PCR-products of 243 bp for *AHK2* and 231 bp for *AHK3* spanning approximately a third of the respective CHASE-domains.

### Used primers are:

pUC19-3-*AHK2*-forward: XhoI - AGGGCATCAGGAAAAGGAGT
pUC19-3-*AHK2*-reverse: XhoI -AvrII- TGTCTGTTTGCTGGCAAGTT

The resulting PCR-product is cloned in antisense orientation in the XhoI site of pUC19-PP (see 3.A.) to yield pUC19-PP-A2rix.

### Step2:

pUC19-3-*AHK3*- forward: AvrII - GGTATCTCGGGGGAGTGTTT
pUCl9-3-*AHK3*-reverse: AvrII - AscI-CGCATCTCATGCTTTCTCAA

The resulting PCR-product is cloned in antisense orientation in the AvrII site of pUC19-PP-A2rix yielding pUC19-PP-A23rix.

### Step3:

The insert downstream of the PYK10 promoter (i.e. the fused AHK2, AHK3 CHASE segments) of pUC19-PF-A23rix is subcloned into pHellsgate2 by PCR-based cloning.

Primers are:
pHellsgate2-3-forward: attB1- AvrII- AGGGCATCAGGAAAAGGAGT
pHellsgate2-3-reverse: attB2- XhoI - CCAAAATCGAGCTTGCTCTC

Introduction of the construct in pHellsgate2 via recombination (PB-reaction) yields pHG-A23rix.

### Step4:

It follows a PCR-based cloning step with BamHI as linkers to reintroduce this RNAi construct into pUC19-PP (see 3.A.) downstream of the PYK10 promoter.
pUC19-31-forward: BamHI-attB1- AvrII- AGGGCATCAGGAAAAGGAGT
pUC19-31-reverse: BamHI-attB2- XhoI - CCAAAATCGAGCTTGCTCTC

The resulted PCR-product is cloned in the BamHI site of pUC19-PP to yield pUC19-PP-A23rix-II.

### Step5:

The insert of pUC19-PP-A23rix-II is subcloned in the SmaI site of the binary vector pGPTV-KAN to yield pPP-A23rix.

### Step6:

*Agrobacterium* harbouring pPP-A23rix are used to transform Arabidopsis using the flower dip vacuum infiltration method. Transgenic plants were selected on media containing kanamycin.

### Evaluation of transgenic plants

For each of the above constructs at least ten different transgenic lines are obtained. Root growth is surveyed in the T1 generation and homozygote T2 plants of the best two clones are analyzed in further detail. Shoot growth and reproductive development of these transgenic lines are not affected. Quantitative comparison of elongation of the primary root and the number of lateral roots 21 days after germination indicate a significant increase in root mass in all cases. In particular, side root formation is enhanced in the transgenic clones compared to wild-type. Irrespective of the construct used all transgenic lines show an increase in side root formation by 30% to 150% and more. These results demonstrate that organ-specific downregulation of the AHK2 and AHK3 genes results in root enhancement without affecting the development of other plant organs.

Within this Example 2 the following references reflecting common knowledge of a person skilled in the art are cited:
Becker et al. (1992) New plant binary vectors with selectable markers located proximal to the left T-DNA border. Plant Mol Biol, 20, 1195-1197.
Kinoshita et al. (2004) One-way control of FWA imprinting in Arabidopsis endosperm by DNA methylation. Science, 303, 521-523.
Koncz, C., Schell, J. (1986) The promoter of the TL-DNA gene 5 controls the tissue-specific expression of chimaeric genes carried by a novel type of Agrobacterium binary vector. Mol. Gen. Genet. 204, 383-396.
Luo M, Bilodeau P, Dennis ES, Peacock WJ, Chaudhury A. (2000) Expression and parent-of-origin effects for FIS2, MEA, and FIE in the endosperm and embryo of developing Arabidopsis seeds. Proc Natl Acad Sci U S A. 97, 10637-10642.
Nitz I, Berkefeld H, Puzio PS, Grundler FM. (2001) Pyk10, a seedling and root specific gene and promoter from Arabidopsis thaliana.Plant Sci. 161, 337-346.
Wesley et al. (2001) Construct design for efficient, effective and high-throughput gene silencing in plants. Plant J, 27, 581-590.

## Claims

1. A transgenic plant, wherein the activity of at least one naturally occurring cytokinin-receptor DNA, RNA and/or protein is selectively suppressed or reduced in a specific target tissue of the plant, and wherein the activity of the naturally occurring cytokinin-receptor DNA, RNA and protein is essentially unaltered in at least one tissue of the plant not being target tissue.

2. The transgenic plant of claim 1, wherein the target tissue is selected from the group consisting of "root tissue, embryo tissue, endosperm tissue, and aleurone tissue".

3. The transgenic plant of claim 1 or claim 2, wherein the activity of the cytokinin-receptor is reduced by inhibiting or reducing the expression of cytokinin-receptor.

4. The transgenic plant of one of the claims 1 to 3, wherein the expression of the cytokinin-receptor is reduced by tissue-specific transcription of foreign RNAi or antisense RNA being under the control of a tissue-specific regulatory element.

5. The transgenic plant of claim 4, wherein the tissue-specific regulatory element is a promotor selected from the group consisting of promotors disclosed in the tables.

6. The transgenic plant of any of the claims 1 to 5, which is naturally not capable of reducing cytokinin-receptor expression tissue-specifically, wherein a foreign DNA sequence is introduced by genetic engineering, which encodes for at least one nucleic acid or at least one protein or peptide being directly or indirectly inhibitory for at least one cytokinin-receptor DNA, RNA and/or protein, wherein the foreign DNA sequence stands under the control of a tissue-specific regulatory element.

7. The transgenic plant of any of the claims 1 to 6 producing seeds of enhanced size and/or producing roots with enhanced branching.

8. Parts, cells, or seeds of a transgenic plant according to one of the claims 1 to 7.

9. Plants or propagating material thereof regenerated from a transgenic plant according to one of the claims 1 to 7.

10. Process for making a transgenic plant or material according to one of the claims 1 to 9, wherein a vector comprising a DNA sequence, which encodes for at least one nucleic acid or at least one protein or peptide being inhibitory for at least one cytokinin-receptor DNA, RNA and/or protein, and which stands under the control of a tissue-specific regulatory element, is introduced in a gene technological manner into cells of a plant naturally not capable of reducing cytokinin-receptor expression tissue-specifically, wherein the cells are transformed.

11. Process according to claim 10, wherein transformed cells are selected and transformed plants are regenerated from the cells.

12. Use of a transgenic plant, wherein the activity of at least one cytokinin-receptor DNA, RNA, and/or protein is reduced, in particular according to one of the claims 1 to 7, for producing seeds of enhanced size, wherein the transgenic plants are cultured under culturing conditions and the preferably mature seeds are harvested.

13. Use of a transgenic plant, wherein the activity of at least one cytokinin-receptor DNA, RNA and/or protein is reduced, in particular according to one of the claims 1 to 7, for producing a live root system with enhanced branching, wherein the transgenic plant is cultured under culturing conditions.

14. Method for enhancing the seed size or the root branching of a plant, wherein the activity of at least one cytokinin-receptor DNA, RNA and/or protein is selectively suppressed or reduced in a specific target tissue of the plant, and wherein the activity of the same cytokinin-receptor is essentially unaltered in tissue not being target tissue.

15. The method of claim 14, wherein the target tissue is selected from the group consisting of "root tissue, embryo tissue, endosperm tissue, and aleurone tissue".

16. The method of claim 14 or claim 15, wherein the activity of the cytokinin-receptor is reduced by inhibiting or reducing the expression of the cytokinin-receptor.

17. The method of one of the claims 14 to 16, wherein the expression of the cytokinin-receptor is inhibited or reduced by tissue-specific expression of foreign RNAi or antisense RNA being under the control of a tissue-specific regulatory element.

18. The method of claim 17, wherein the tissue-specific regulatory element is a promotor selected from the group consisting of the promotors disclosed in table 1.

19. The method of any of the claims 14 to 18, wherein the plant is naturally not capable of reducing the expression of at least one cytokinin-receptor tissue-specifically, wherein a foreign DNA sequence is introduced by genetic engineering, which encodes for at least one nucleic acid or at least one protein or peptide being inhibitory for the cytokinin-receptor DNA, RNA and/or protein, and wherein the foreign DNA sequence stands under the control of a tissue-specific regulatory element.

20. The method of any of the claims 14 to 19, wherein the plant produces seeds of enhanced size and/or produces roots with enhanced branching.

21. The method of any of the claims 14 to 20, wherein the plant is cultured under culturing conditions.

22. A method for making seeds of enhanced size, wherein the transgenic plant, parts thereof, or seeds according to any of the claims 1 bis 9 are cultured under culturing conditions and preferably mature seeds being produced thereby are harvested.

23. Seeds obtainable by a method of any of the claims 14 to 22.

24. Use of an isolated peptide or protein comprising the sequence CKnVLTxPF, wherein n is A or G and x is any amino acid, or comprising the sequence LGVxxTm, wherein m is F or Y and x has then meaning as above, or of an isolated nucleic acid coding for such protein or peptide for making a cytokinin-receptor inhibitor, wherein a substance, preferably a small molecule, antibody, peptide, protein, or inhibitory nucleic acid is constructed by means of molecular modelling, which binds to said amino acid sequence or to said nucleic acid sequence, or wherein such substance is selected from a substance pool after subjecting the substances of the substance pool to a binding assay detecting binding to said amino acid sequence or nucleic acid sequence and having detected binding substances.

25. Use of an isolated peptide or protein comprising the sequence CKnVLTxPF, wherein n is A or G and x is any amino acid, or comprising the sequence LGVxxTm, wherein m is F or Y and x has then meaning as above, or of an isolated nucleic acid coding for such protein or peptide for identifying a cytokinin-receptor in a plant, wherein nucleic acids or proteins naturally occurring in the plant are screened for partial sequences being identical with one of said sequences and wherein a nucleic acid or a protein is selected, which comprises a partial sequence with at least 80%, preferably 100%, identity with one of said sequences, as being cytokinin-receptor-functional.
